# EUROPEAN PATENT APPLICATION

(11) **EP 2 100 961 A1**
(43) Date of publication of application: **16.09.2009**
(21) Application number: 08004005.8
(22) Date of filing: 04.03.2008
(51) Int. Cl.: C12N 15/82, A01H 5/00

(54) **Method of protease production in plants**

(71) Applicant: Icon Genetics GmbH, 80333 München (DE)
(72) Inventor: Kandzia, Romy, 06130 Halle (DE); Engler, Carola, 06120 Halle (DE); Marillonnet, Sylvestre, 06126 Halle (DE); Klimyuk, Victor, 06114 Halle (DE); Gleba, Yuri, 06114 Halle (DE)
(74) Representative: Blodig, Wolfgang

(57) **Abstract**

A process of producing a protease in a plant or in plant cells, comprising
(a) providing a plant comprising a heterologous nucleotide sequence comprising a coding sequence encoding a fusion protein, said fusion protein comprising: an apoplast or plastid signal peptide; a SUMO protein or a derivative of a SUMO protein; and a zymogen of said protease, and
(b) expressing said fusion protein.

## Description

### FIELD OF THE INVENTION

The present invention relates to a process for the production of a protease of interest in a plant or in plant cells and protease obtained thereby. Further, the invention relates to a nucleotide sequence and a vector used for the process of the invention and to a transgenic plant or plant cells containing the nucleotide sequence or vector. Further, the invention relates to the use of a SUMO protein or a derivative thereof for expressing a protease of interest in a plant or in plant cells. vectors for this process and to plants or plant cells transformed therewith.

### BACKGROUND OF THE INVENTION

Recombinant protein production in plant systems has been very successful for many different products, covering proteins with industrial applications, food and feed additives, animal health products and human pharmaceuticals, such as antigens and immune response proteins.

There are many comprehensive reviews describing the field (Hood & Jilka, 1999, Curr. Opin. Biotech., 10:382-386; Doran, 2000, Curr. Opin. Biotech., 11:199-204; Daniell, et al., 2001 Trends Plant Sci., 6:219-226; Larrick & Thomas, 2001, Curr. Opin. Biotech., 12:411-418; Klimyuk et al., 2005, in: Modern Biopharmaceuticals, ed. J. Knaeblein, WILEY-VCH, Weinheim, 893-917; Gleba et al., 2007, Curr Opin. Biotechnol., 18, 134-141). Plants have been considered as a low-cost production system for proteins, that is significantly cheaper in comparison with bacterial, yeast, insect and mammalian cell-based production systems. The available data in this regard confirm the above said.

However, a challenge still exists when a commercially applicable expression level of cytotoxic proteins such as proteases has to be acheaved. Proteases are used in many different commercial applications, including pharmaceutical and laboratory uses. The sources of proteases are usually either animal tissues (e.g. bovine and porcine pancreas for trypsin and chymotrypsin production) or bacteria (e.g. strains of Bacillus for subtilisin and thermolysin production). In the case of animal tissue as the source of proteases, potential cross-contamination by undesirable components (e.g. prions or other infectious agents) produced by the animal cells must be taken care of. Bacterial production of many proteases is rather restricted due to their low yield that in many cases is well below commercially viable levels.

Production of chymotrypsin (US2006015971) and trypsin (US6087558) in plants (corn grain) was previously described. This method provides a plant source of recombinant proteases with several of the advantages of a plant production host. In a prior art plant expression system for trypsin (Woodard et al., 2003, Biotechnol. Appl. Biochem., 38, 123-130), trypsin was targeted in the zymogen form to the cell wall in corn seeds using an embryo-preferred promoter in transgenic maize plants. Several other promoter or subcellular target sites gave inferior expression yields. Seeds were considered ideal for trypsin expression due to their content of trypsin inhibitor that may minimize detrimental effects of trypsin on the host plant.

However, it follows from the published data (Woodard et al., 2003, Biotechnol. Appl. Biochem., 38, 123-130) that the highest expression level of trypsin obtained in corn seeds is rather low (ca. 58 mg/kg of corn grain), which inevitably affects the market cost of recombinant trypsin. Indeed, the cost of plant-derived recombinant trypsin is significantly higher in comparison with that from traditional sources. This is not surprising as the cost of recombinant protein production (including downstream processing) is reversely dependent on the expression level of the protein. For example, extraction of beta-glucuronidase (GUS) from transgenic corn seeds accounts for 94% of the production cost (Evangelista et al., 1998, Biotechnol. Prog., 14:607-614). The calculations were done for transgenic corn containing 0.015% of recombinant GUS. It was stated that increase in recombinant GUS expression level up to 0.08% (4-5 folds) significantly improves the process economics. Also, the production of recombinant protease in agriculturally important plant that is commonly used in feed /food chains creates additional biosafety risks of cross-contaminating non-transgenic seed stock.

Departing from the prior art, it is the problem of the invention to provide a plant expression system and production process for proteases that gives high yield. It is a further object of the invention to provide a plant expression system for proteases that avoids contamination of food, feed or seed stock intended for human or animal consumption by transgenic plant material. It is a further object of the invention to provide a process of producing a protease of interest in plants or plant cells, which addresses the problems associated with toxicity of said protease to plant cells expressing the protease and offers an economic way of the protease production in plants.

### GENERAL DESCRIPTION OF THE INVENTION

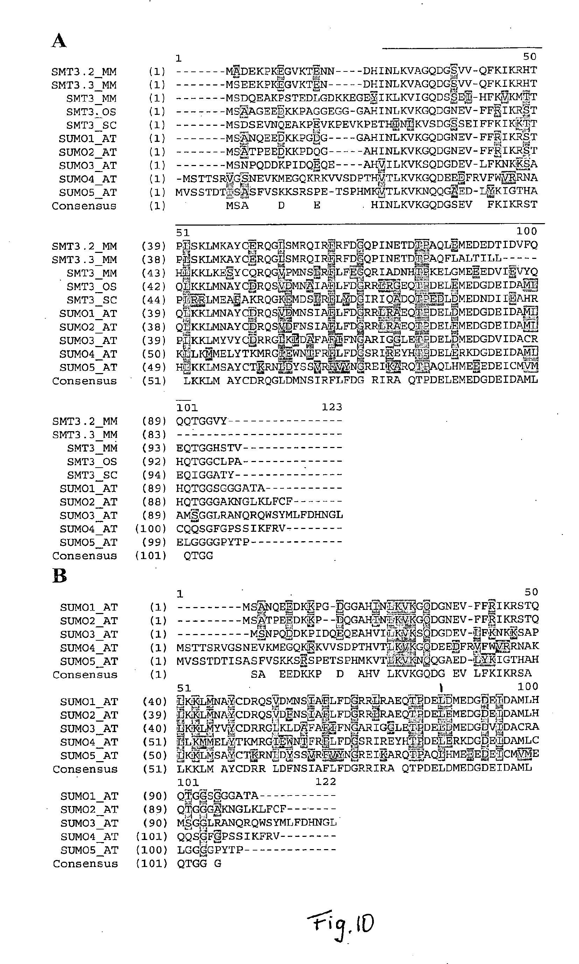

The present invention provides a process of producing a protease in a plant or in plant cells, comprising
(i) providing a plant comprising a nucleotide sequence comprising a coding sequence encoding a fusion protein, said fusion protein comprising an apoplast or plastid signal peptide, a SUMO protein or derivative of a SUMO protein, and a zymogen of said protease, and
(ii) expressing said fusion protein.
   The present invention further provides a process of producing a protease in a plant or in plant cells, comprising providing a plant with a replicon comprising a coding sequence encoding a fusion protein, said fusion protein comprising an apoplast or plastid signal peptide, a SUMO protein or a C-terminal domain of a SUMO protein, and a zymogen of said protease.
   The invention also provides a, preferably isolated, vector or nucleotide sequence comprising a coding sequence encoding a fusion protein, said fusion protein comprising an apoplast or plastid signal peptide, a SUMO protein or derivative of a SUMO protein, and a zymogen of said protease.
   The present invention also provides a transgenic plant or plant cells for expressing a protease or a fusion protein comprising said protease, said plant comprising a nucleotide sequence containing a promoter active in vegetative tissue and, downstream of said promoter and operably linked thereto, a coding sequence encoding a fusion protein comprising a plastid or apoplast signal peptide, a SUMO protein or a derivative thereof, and a zymogen of said protease.
   The present invention also provides a use of a SUMO protein or a derivative of a SUMO protein for expressing a protease or a zymogen of a protease in a plant or in plant cells.
   Transgenic corns seeds used in the prior art for protease production in plants are a problematic production system, since it is almost impossible to avoid contamination of non-GM corn seeds with traces of transgenic corn seeds in industrial scale agriculture. Further, the expression yield obtained for trypsin in corn seeds is not satisfactory, resulting in high costs of trypsin produced by this method. The inventors of the present invention have surprisingly found that it is possible to obtain a high expression yield of proteases in plant leaves although protease inhibitors (such as trypsin inhibitor) reducing a proteolytic degradation of plant host tissue expressing the protease were not expected to a significant extent in leaf tissue. Contrary to what could be expected from Woodard et al. (2003, Biotechnol. Appl. Biochem., 38, 123-130), vegetative (or green) tissue such as leaf material has been found to be an ideal tissue for expressing proteases in plants in a high yield.
   The process of the invention is not limited to maize but can be performed in plants such as Nicotiana species that are not part of the human food chain. Further, it is not necessary to harvest seeds or transgenic plants for isolating the protease. Instead, the invention can be performed by harvesting plants having expressed protease before seeds have reached a viable growth state. Thereby, contamination of non-GM corn seeds by transgenic seeds can be effectively avoided. In one embodiment, the invention allows it to avoid the use of transgenic plants by using transient expression of the protease of interest. In contrast, transient expression is difficult to practice if a protease is expressed in corn seeds as described by Woodard et al.
   The process of the invention can be performed by transient expression or using transgenic plants. Transgenic plants for the invention contain in a nuclear genome the heterologous nucleotide sequence of the invention. In a transient expression system, said nucleotide sequence is provided to a plant, whereby production of said protease may be triggered by the provision of said nucleotide sequence to a plant. The nucleotide sequence is typically not incorparated into the genome of the plant host when the process of the invention is performed by transient expression. In any event, in a transient expression process, plants or plant cells having incorporated the nucleotide sequence into their genome are not selected, e.g. by using an antibiotic resistance marker, from plants or cells not having the nucleotide sequence incorporated into the genome. As a consequence, plants or plant cells having incorporated the nucleotide sequence of the invention into their genome are not produced to a significant extent, whereby transmission of the nucleotide sequence of the invention to progeny plants and seeds is very unlikely. If the process of the invention is carried out by transient expression, the nucleotide sequence of the invention may be provided to a plant having reached a desired growth state.
   Said nucleotide sequence of the invention is heterologous, since it does not naturally occur in genome of the plant, or cells thereof, used in the process of the invention.
   The nucleotide sequence of the invention comprises the coding sequence of the invention. The coding sequence encodes the fusion protein of the invention. In addition to said coding sequence, the nucleotide sequence of the invention may further have genetic elements for expressing said coding sequence into said fusion protein. Examples of such genetic elements are a promoter active in said plant and a transcription termination region. Said promoter is operably linked to said coding sequence such that expression of the coding sequence is under the control of the promoter. The promoter may be a constitutive promoter active in said plant such as the CaMV 35S promoter. Alternatively, said promoter may be an inducible promoter so that production of said protease can be induced at will, such as at a desired growth state of the plant. In a further alternative, said nucleotide sequence may be incorporated into a chromosome of said plant such that expression of said coding sequence is possible under the transcriptional control of a native host promoter as described in WO 02/46440.
   In one embodiment, said nucleotide sequence is or encodes a replicon comprising said coding sequence encoding said fusion protein. Herein, a replicon is a nucleic acid capable of replicating independently from the plant nuclear replication machinery. For this purpose, said replicon has an origin of replication that can be recognized by a nucleic acid polymerase that is present in or that is provided to cells of said plant. In one embodiment, said replicon is a viral replicon. The viral replicon may be a DNA viral replicon or an RNA viral replicon. In an advantageous case, the replicon is an RNA viral replicon. Viral RNA replicons generally use viral polymerases for replicating the replicon; polymerases native to the plant host generally cannot replicate viral RNA replicons. RNA viral replicons further use an origin of replication that is not recognized by native plant polymerases. The viral polymerase may be encoded on the RNA replicon or may be provided in trans from a separate vector or from a transgene encoding such viral polymerase whereby the transgene is incorporated into a nuclear or organellar genome of the plant.
   Said nucleotide sequence may be or may encode an RNA replicon such as a viral RNA replicon. Said viral RNA replicon may use the replication and expression machinery of a natural plant RNA virus. Suitable RNA viruses from which RNA replicons of the invention may be built on are, for example, positive-sense single-stranded plant RNA viruses. Examples of such plant RNA viruses are tobamoviruses such tobacco mosaic virus, crucifer-infecting tobamovirus, or turnip vein clearing virus. These viruses and their use for expressing a protein of interest in plants are known (see below). Thus, said RNA replicon may encode an RNA-dependent RNA polymerase ("replicase") capable of replicating said RNA replicon. Further, the RNA replicon will contain an origin of replication that is recognized by the replicase.
   There are various ways how a plant or plant cells can be provided with the replicon of the invention. In one embodiment, the nucleotide sequence of the invention is said replicon, and the plant or plant cells are infected directly with the replicon.
   In another embodiment, the plant or plant cells are provided with a nucleotide sequence encoding said replicon. If said replicon is an RNA replicon, the nucleotide sequence may be a DNA nucleotide sequence encoding said RNA replicon. The DNA nucleotide sequence may have a promoter for producing said RNA replicon by transcription of a portion of said nucleotide sequence encoding said replicon, e.g. by a native RNA polymerase of the plant or plant cell.
   A DNA nucleotide sequence encoding an RNA replicon may be incorporated into the nuclear genome of a plant or plant cells, whereby a transgenic plant or plant cells, respectively, are produced. The promoter present in the DNA nucleotide sequence may be an inducible promoter. In this way, formation of the replicon from which said fusion protein is expressed can be triggered at will by inducing the inducible promoter. Suitable inducible promoters are known in the art. Said inducible promoter may be part of an alcohol inducible system as described in example 4. Measures to suppress the consequences of any leaky expression by an inducible promoter system may be taken, e.g. as described in WO 2007/137788 that is incorporated herein in its entirety. This embodiment may be used together with transgenic plants containing said nucleotide sequence integrated into a nuclear or organellar chromosome.
   In a transient expression method of the invention, a plant may be provided with a replicon. Similarly as described above, said replicon may be a DNA or an RNA replicon such as a viral DNA replicon or a viral RNA replicon. Cells of said plant may be provided with said replicons directly, such as by infecting said plant with said replicon or by particle bombardment using particles coated with said replicon. Alternatively, a plant may be provided with a replicon indirectly, such as by Agrobacterium-mediated transfection using Agrobacteria containing Ti plasmids containing T-DNA comprising a nucleotide sequence encoding said replicon. After having entered cells of said plant, the DNA or RNA replicon can be activated from said T-DNA by transcription by a DNA-dependent polymerase such as a DNA-dependent polymerase that is native to said plant. For this purpose, the T-DNA typically contains a promoter upstream of the nucleic acid encoding the replicon. After formation of the replicon in cells of said plant, the replicon replicates and expresses said coding sequence for producing the fusion protein of the invention.
   In an advantageous transient expression method, the replicon is an RNA replicon and said plant is provided with said RNA replicon by transforming said plant or said plant cells with a DNA vector as a DNA nucleotide sequence encoding said RNA replicon. Alternatively, said plant or said plant cells are provided with two or more DNA vectors encoding together said RNA replicon, whereby a DNA nucleotide sequence encoding an RNA replicon may be generated inside cells of said plant e.g. by site-specific DNA recombination as described in WO 02/88369. After recombination, the RNA replicon may be formed by transcription involving a native plant host RNA polmyerase and a promoter present on the DNA nucleotide sequence.
   The coding sequence of the invention encodes the fusion protein of the invention, said fusion protein comprising at least the following three fusion protein segments: (i) an apoplast or plastid signal peptide, (ii) a SUMO protein or a derivative of a SUMO protein, and (iii) a zymogen of said protease. These elements of said fusion protein may be arranged in different orders. However, the signal peptide is preferably placed at the N-terminal end of the fusion protein in order to be functional for targeting the fusion protein to the apoplast or into the plastids. The zymogen of said protease may be placed at the C-terminus of the fusion protein, which allows its easy separation from the remainder of the fusion protein by a proteolytic cleavage at a single peptide bond. Thus, in one embodiment, the order of the essential fusion protein segments (i) to (iii) may be, in N-terminal to C-terminal direction, as listed in claim 1.
   The fusion protein of the invention may further contain a polypeptide segment usable as a purification tag for facilitating the purification of the fusion protein or the protease of interest. The purification tag may be located at an internal position of said fusion protein such as on the C-terminal side of the signal peptide and on the N-terminal side of the zymogen of the protease. However, other orders are also possible e.g. placing the purification tag at the C-terminus of the SUMO protein. Purification tags that can be used for practicing this invention include, but are not limited to: FLAG tag, polyhistidine tags, polyarginine tags, influenza virus HA tag,GST-tag, protein A tag, maltose binding protein (MBP), S-tag, the AviD tag, etc.
   The protease to be produced according to the invenion may be any protease that is naturally expressed as a zymogen that is activated by proteolytic cleavage to produce the active protease. Such proteases are known in the art. Chymotrypsin and trypsin are examples of proteins to be produced by the invention. The zymogens of chymotrypsin and trypsin are chymotrypsinogen and trypsinogen, respectively. Other examples may include but are not limited to precursors (zymogens) of barley protease EPB2 indicated for celiac sprue treatment (Mikkonen et al., 1996, Plant Mol Biol., 31, 239-254; Vora et al., 2007, Biotechnol Bioeng., 98, 177-185.), elastase, caspases, carboxypeptidase A, thrombin and other proteases.
   Plastid signal peptides (also referred to as "plastid transit peptides" in the art) for targeting proteins into plastids are known in the art. Examples of plastig signal peptides are found in WO 2004/101797. Signal peptides for targeting proteins into the secretory pathway and into the apoplast are also known from general knowledge. The signal peptides described in WO 02/101006 may be used for targeting the fusion protein to the apoplast.
   Said fustion protein further comprises a SUMO protein or a derivative of a SUMO protein. SUMO proteins usable in the present invention are SUMO proteins that were used in the prior art in bacterial protein expression systems, cf. Butt et al., 2005, Protein Expr Purif., 43, 1-9; Marblestone et al., 2006, Protein Sci., 15, 182-189; Su et al., 2006, Protein Pept. Lett., 13, 785-792; Weeks et al., 2007, Protein Expr Purif., 53, 40-50; US2004018591; EP1654379).
   SUMO (small ubiquitin-like modifier) is a member of the superfamily of ubiquitin-like polypeptides (Melchior,F., 2000, Annu. Rev. Cell. Dev. Biol., 16, 591-696; Schwartz & Hochstrasser, 2003, Trends Biochem. Sci., 28, 321-328; Dohmen, RJ., 2004, Biochim. Biophys. Acta, 1695, 113-131; Hay, RT., 2007, Trends Cell Biol., 17, 370-376). All SUMO proteins contain a ubiquitin domain (outlined in Fig. 10A) and are about 100 amino acid residues in length (usually within the range of 90 and 115). Alignments of different SUMO proteins from different organisms including plant SUMO proteins are shown in Figure 10.
   In higher plants several genes were identified that encode different SUMO proteins. The genes are designated SUMO1, SUMO2, SUMO3, SUMO4, SUMO5, SUMO6, SUMO7, SUMO8 and SUMO9 in Arabidopsis, but only four of them (SUMO1, SUMO2, SUMO3 and SUMO5) were found to be transcriptionally active (Kurepa et al., 2003, J. Biol. Chem., 278, 6862-6872). Among these SUMO proteins, the fusion with SUMO1 generally gives the highest expression levels for protein of interest in the present invention. In the present invention, a SUMO protein known from an organism such as a plant, an animal or yeast may be used, whereby plant SUMO proteins are generally preferred.
   Alternatively, derivatives of a natural SUMO protein may be used. A derivative of a SUMO protein herein comprises, in one embodiment, at least 50 contiguous amino acid residues, in another embodiment at least 60 contiguous amino acid residues, in a further embodiment at least 70 contiguous amino acid residues, and in a still further embodiment at least 80 contiguous amino acid residues.
   A SUMO protein-derivative according to the invention is characterized by comprising the typical consensus sequence of a SUMO protein. Such consensus sequence can be determined by making sequence alignments of known SUMO proteins. The consensus sequence defines the a specific amino acid residue or a selection of specific amino acid residues for certain amino acid residue positions, whereas any desired amino acid residue can be chosen at other positions with little influence on the expression properties of the protease to be expressed according to the invention. Suitable consensus sequences can be defined at varying degrees of specificity. In the broadest sense, the derivative of a SUMO protein has the consensus sequence: -L/F/M-X₁₉-F/I-X₃-G/D-X₇-T/S-P/A-.

In other embodiments of the invention, a derivative of a SUMO protein has any one of the following amino acid sequences:
-L/F/M-X₁₉-F/I-X₃-G/D-X₇-T-P-;
-L-(X)₁₉-F-X₃-G-X₇-T-P-;
-L-X₁₉-F-X₃-G-X₇-T-P-X₁₈-G-G-;
-L/I-X-V/L-Xₐ-L-X₁₉-F-X₃-G-X₇-T-P-;
-L-X-K/R-X₁₇-F-X₃-G-X₇-T-P-;
-L-X-K/R-UM-X₁₆-F-X₃-G-X₇-T-P-;
-L-X-K/R-UM-M-X₁₅-F-X₃-G-X₇-T-P-;
-L-X-K/R-L/M-M-X₁₅-F-X₃-G-X₇-T-P-X₁₈-G-G-;
-L-X-K/R-K/R-M-X₁₅-F-X₃-G-X₇-T-P-X₂-L-;
-L-X-K/R-L/M-M-X₁₅-F-X₃-G-X₇-T-P-X₂-L-D/E-X₂-D/E-;
-L-X-K/R-UM-M-X₁₅-F-X₃-G-X₇-T-P-X₂-L-D/E-X₂-D/E-X₃-I-D/E-;
-L-X-K/R-L/M-M-X₁₅-F-X₃-G-X₇-T-P-X₂-L-D/E-X₂-D/E-X₃-I-D/E-X₆-G-G-;
-L/I-X-V/L-Xₐ-L-X-K/R-L/M-M-X₁₅-F-X₃-G-X₇-T-P-X₂-L-D/E-X₂-D/E-X₃-I-D/E-X₆-G-G-;
-L-K-V-K-X_{b}-L-X₁₉-F-X₃-G-X₇-T-P-;
-L-K-V-K-X_{b}-L-X-K-X-M-X₁₅-F-X₃-G-X₇-T-P-;
-L-K-V-K-X_{b}-L-X-K-X-M-X₂-Y-X₁₂-F-X₃-G-X₇-T-P-;
-L-K-V-K-X_{b}-L-X-K-X-M-X₂-Y-X₁₂-F-X₃-G-X₇-T-P-X₂-L-;
-L-K-V-K-X_{b}-L-X-K-X-M-X₂-Y-X₁₂-F-X₃-G-X₇-T-P-X₂-L-X₁₅-G-G-;
-L-K-V-K-X_{b}-L-X-K-X-M-X₂-Y-X₁₂-F-X₃-G-X₇-T-P-X₂-L-X₇-I-;
-L-K-V-K-X_{b}-L-X-K-X-M-X₂-Y-X₁₂-F-X₃-G-X₇-T-P-X₂-L-X₅-X-I-;
-L-K-V-K-X_{b}-L-X-K-X-M-X₂-Y-X₁₂-F-X₃-G-X₇-T-P-X₂-L-X₇-I-X₇-G-G-;
-L-K-V-K-X-Q-X_{c}-L-X-K-X-M-X₂-Y-X₁₂-F-X₃-G-X₇-T-P-X₂-L-X₇-I-X₇-G-G-;
-L-K-V-K-X_{b}-L-L/K-K-L/M-M-X₂-Y-X₁₂-F-X₃-G-X₇-T-P-X₂-L-X₇-I-X₇-G-G-;
-L-K-V-K-X-Q-X_{c}-L-L/K-K-L/M-M-X₂-Y-X₁₂-F-X₃-G-X₇-T-P-X₂-L-X₇-I-X₇-G-G-;
-L-X-K/R-L-X₁₆-F-X₃-G-X₇-T-P-;
-L-X-K/R-L-M-X₁₅-F-X₃-G-X₇-T-P-;
-L-X-K/R-L-M-X₅-R/K-Q/R-X₈-F-X₃-G-X₇-T-P-;
-L-X-K/R-L-M-X₅-R-Q-X₈-F-X₃-G-X₇-T-P-;
-L-X-K/R-L-M-X₅-R-Q-X₈-F-X₃-G-X₇-T-P-X₂-L-X-M-;
-L-X-K/R-L-M-X₅-R-Q-X₃-M-X₄-F-L-X₂-G-X₇-T-P-X₂-L-X-M-;
-L-X-K/R-L-M-X₅-R-Q-X₈-F-L-X₂-G-X₇-T-P-X₂-L-X-M-E-X₄-I-X₇-G-G-:
-L-X-K/R-L-M-X₅-R-Q-X₈-F-L-X₂-G-X-R-X₅-T-P-X₂-L-X-M-E-X₄-I-X₇-G-G-:

In the above sequences of a SUMO protein derivative of the invention, the amino acid consensus sequences are given in N-terminal to C-terminal direction;
a is an integer of 17 or 18;
b is an integer of 16 or 17;
c is an integer of 14 or 15;
   each letter stands for an amino acid residue;
   X stands for any amino acid residue;
   letters other than X stand for amino acid residues in the standard one-letter code;
   a numerical subscript to a letter indicates that the amino acid residue defined by said letter is present contiguously and connected by peptide bonds as many times as indicated by the numerical value of the subscript;
   "-" stands for a peptide bond connecting adjacent amino acid residues; and
   "/" indicates that the amino acid position defined by two consecutive "-" can be occupied by any of the amino acid residues defined by letters separated by "/".

After having expressed the fusion protein of the invention from said coding sequence, said protease or said zymogen or a fusion protein comprising said zymogen can be isolated from vegetative tissue such as from leaf tissue of said plant and purified according to standard method of protein purification. If the isolated protein is not the activated protease but the zymogen of the protease or a fusion protein comprising said zymogen, the active protease may be generated from said zymogen or from said fusion protein by proteolytic cleavage. Said proteolytic cleavage may be achieved by a protease recognizing the cleavage site of said zymogen. If the isolated protein is or comprises trypsinogen, the protease enterokinase may be used for activating trypsin from trypsinogen. Chymotrypsin may be activated from chymotrypsinogen by trypsin.

The present invention can be performed with any plant or cells thereof. It is preferred that the invention is performed with plants. Among plants, higher plants are preferred. Among higher plants, the invention may be performed with monocot or with dicot plants. Plants that are not part of the humn food chain are preferred. Examples of plants that may be used in the invention are Nicotiana species such as *Nicotiana benthamiana* and *Nicotiana tabacum.*

### ADVANTAGEOUS EMBODIMENTS

A process of producing a protease in a plant or in plant cells, comprising
(a) providing a plant comprising a heterologous nucleotide sequence comprising a coding sequence encoding a fusion protein, said fusion protein comprising:
   (i) an apoplast or plastid signal peptide;
   (ii) a SUMO protein or a derivative of a SUMO protein; and
   (iii) a zymogen of said protease, and
(b) expressing said fusion protein, said derivative of a SUMO protein comprising the consensus sequence -L/F/M-X₁₉-F/I-X₃-G/D-X₇-T/S-P/A-.

A process of producing a protease in a plant or in plant cells, comprising
(a) providing a plant comprising, on a nuclear chromosome, a heterologous nucleotide sequence comprising a coding sequence encoding a fusion protein, said fusion protein comprising:
   (i) an apoplast or plastid signal peptide;
   (ii) a SUMO protein or a derivative of a SUMO protein; and
   (iii) a zymogen of said protease, and
(b) expressing said fusion protein,
wherein
said nucleotide sequence comprising an inducible promoter upstream of said coding sequence and operably linked to said coding sequence, and
said derivative of a SUMO protein comprising the consensus sequence -L/F/M-X₁₉-F/I-X₃-G/D-X₇-T/S-P/A-.

A process of producing a protease in a plant or in plant cells, comprising providing a plant with an RNA replicon comprising a coding sequence encoding a fusion protein, said fusion protein comprising:
(i) an apoplast or plastid signal peptide;
(ii) a SUMO protein or a derivative of a SUMO protein; and
(iii) a zymogen of said protease;
   wherein said plant is provided with said RNA replicon by transforming said plant with a DNA vector encoding said RNA vector; and said derivative of a SUMO protein comprises the consensus sequence -L/F/M-X₁₉-F/I-X₃-G/D-X₇-T/S-P/A-. Said DNA vector may contain Agrobacterial T-DNA encoding said RNA replicon and having a promoter for generating said RNA replicon in cells of said plant by transcription.

A process of producing a protease of interest in a plant, comprising:
(A) providing a plant comprising:
   a heterologous nucleotide sequence encoding an RNA replicon and comprising an inducible promoter operably linked to a sequence encoding said RNA replicon;
   said RNA replicon not encoding a protein providing for cell-to-cell movement of said RNA replicon in said plant;
   said RNA replicon encoding a polymerase being adapted for replicating said RNA replicon; said RNA replicon comprising a coding sequence encoding a fusion protein, said fusion protein comprising an apoplast or plastid signal peptide, a SUMO protein or a derivative of a SUMO protein, and a zymogen of said protease; and
(B) inducing, in said plant or plant cells of step (A), said inducible promoter, thereby producing said protease or a fusion protein comprising said protease of interest in said plant or in cells of said plant.

Optionally, said plant may comprise a second heterologous nucleotide sequence comprising a nucleotide sequence encoding a protein enabling cell-to-cell movement of said RNA replicon, wherein said second heterologous nucleotide sequence comprises a second inducible promoter operably linked to said nucleotide sequence encoding said protein enabling cell-to-cell movement of said RNA replicon; said inducible promoter and said second indubible promoter may be the same types of inducible promoters such as promoters of an alcohol inducible system.

Other embodiments described herein may be combined with the above advantageous embodiments.

### BRIEF DESCRIPTION OF THE FIGURES

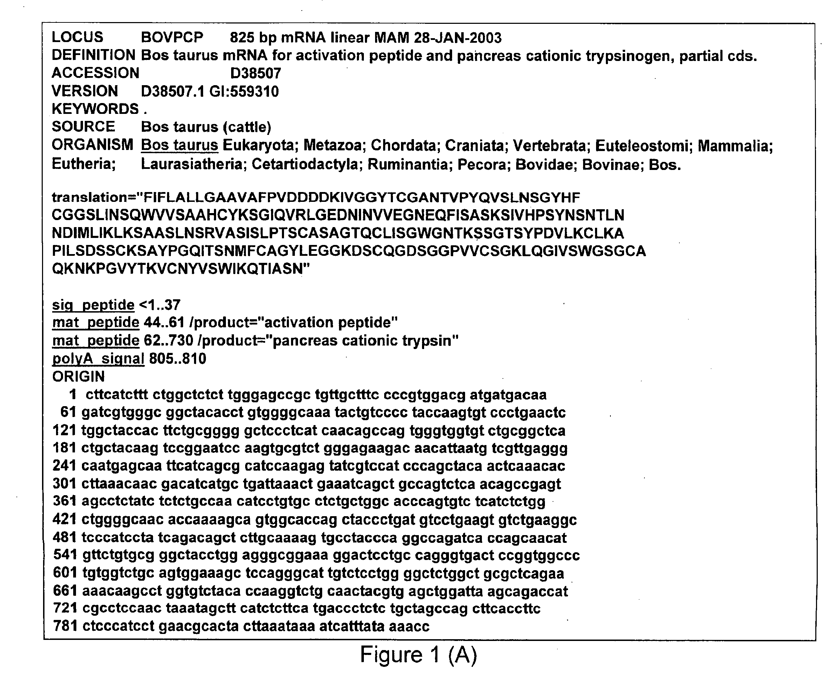

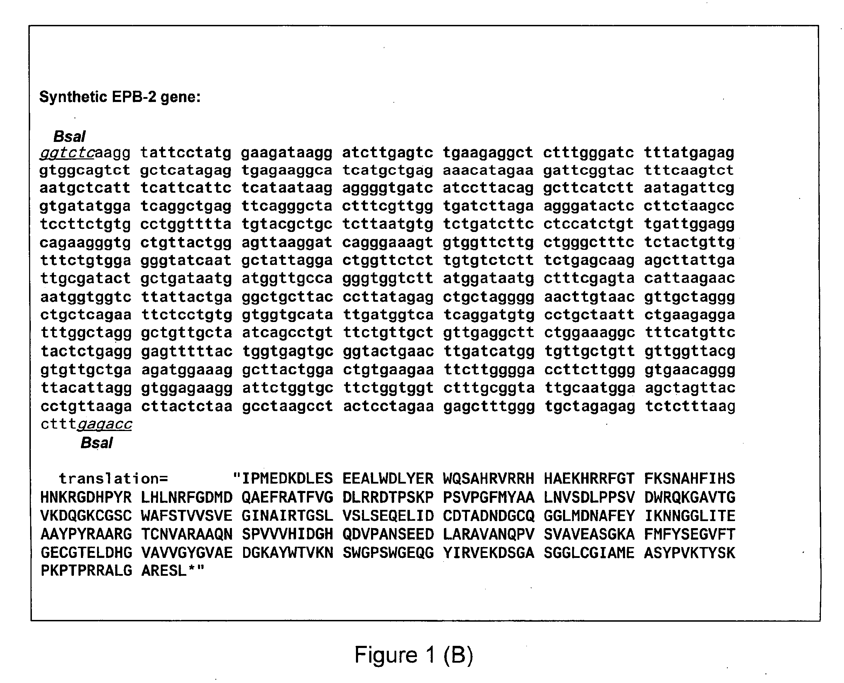

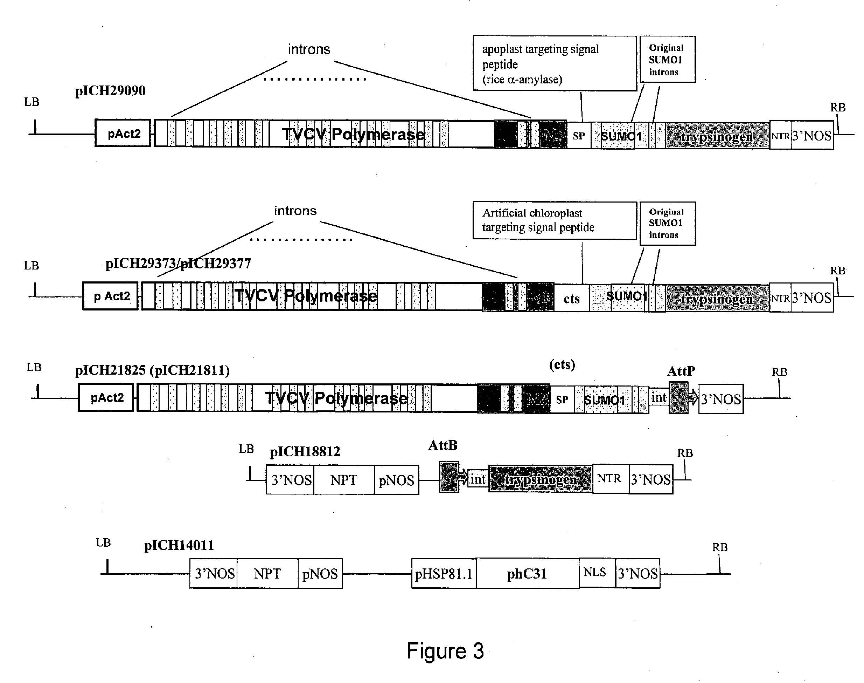

Fig. 1 shows cDNA and protein sequences for (A) bovine pancreas cationic pretrypsinogen and (B) codon-optimised cDNA (synthesised by GENEART AG, Regensburg, Germany) and protein sequences of barley EPB-2 protease precursor. Coding sequences for EPB2 protease precursor are shown in bold. Sequences recognized by site-specific restriction enzyme *Bsa*/ are shown in *italic* and underlined. The protein sequence in Fig. 1(A) is SEQ ID NO: 4. The nucleic acid in Fig. 1(A) is SEQ ID NO: 5. The nucleic acid in Fig. 1(B) is SEQ ID NO: 6. The protein sequence in Fig. 1(B) is SEQ ID NO: 7.

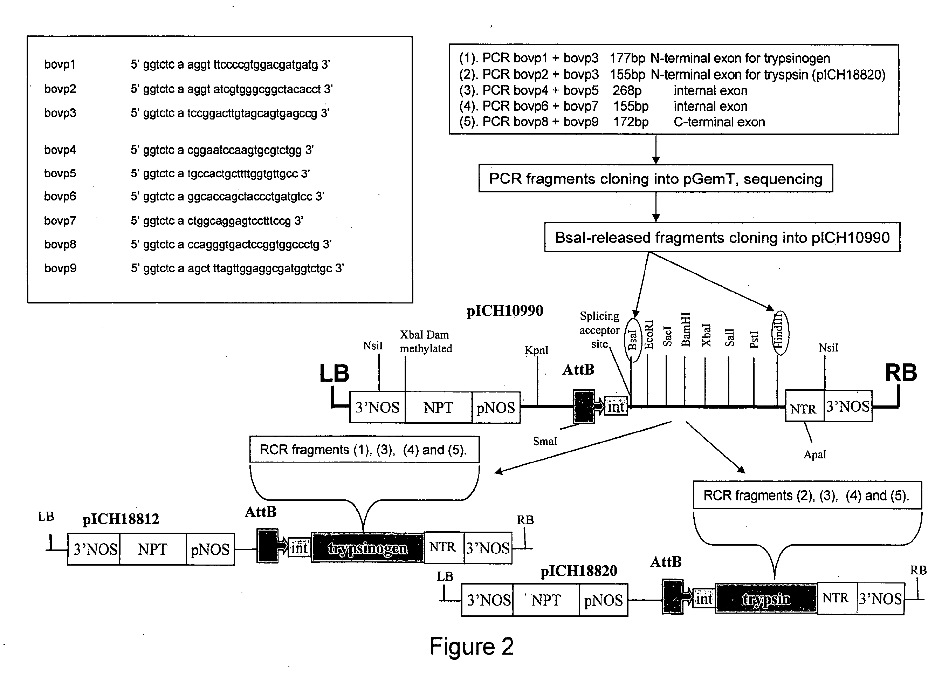

Fig. 2 depicts the cloning strategy for trypsinogen and trypsin genes.
   NTR - viral 3' non-translated region; 3' NOS - transcription termination region of nopaline synthase gene; pNOS - promoter of nopaline synthase; NPTII - neomycin phosphotransferase II gene, AttB - recombination site recognised by site-specific integrase phC31. The sequences of primers bovp1 to bovp9 are shown. Primers bovp1 to bovp9 are SEQ ID NOs: 8 to 16, respectively.0
Fig. 3 depicts T-DNA regions of the binary vectors pICH29090, pICH29373 (pICH29377 - the same as pICH29373, but different clone), pICH21825, pICH21811, pICH18812 and 14011. pAct2 - transcription promoter of Arabidopsis ACTIN2 gene; TVCV polymerase - RNA-dependent RNA polymerase of Turnip Vein Clearing Virus with introns indicated by dotted portions; MP - viral movement protein with introns indicated by dotted portions; NTR - viral 3' non-translated region; 3' NOS - transcription termination region of nopaline synthase gene; SUMO1 - coding sequence with introns (indicated by dotted portions) of Arabidopsis SUMO1 gene; pNOS - promoter of nopaline synthase gene; pHSP81.1 - promoter of the gene for Arabidopsis heat-shock protein HSP81.1; phC31 - site-specific integrase of phage C31; NLS - nuclear localization signal; AttP and AttB - recombination sites recognised by site-specific integrase phC31; SP - apoplast targeting signal peptide (rice amylose); dotted segments stand for introns.

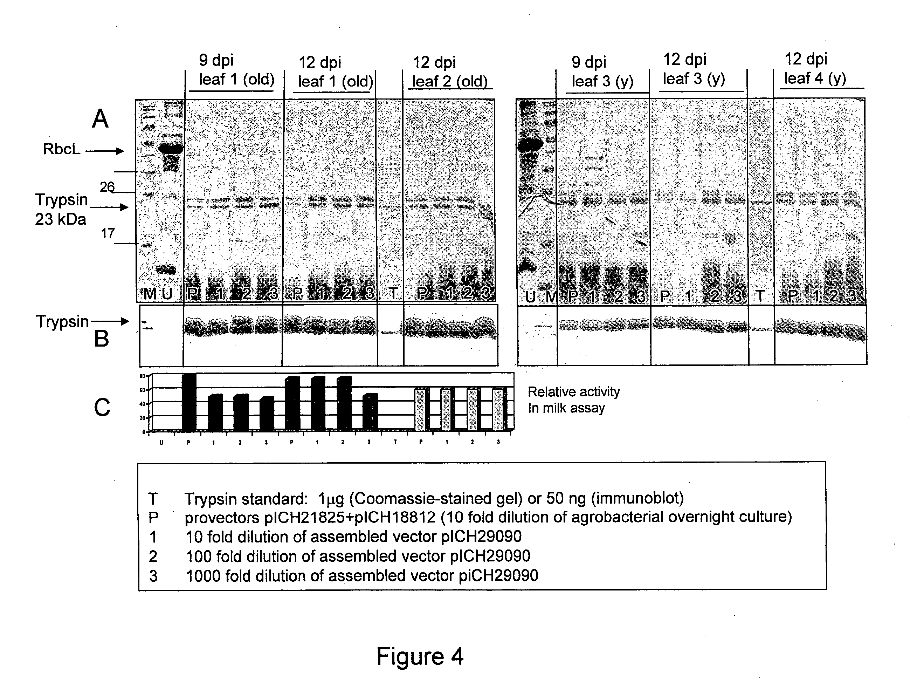

Fig. 4 shows results of expression of apoplast-targeted recombinant SUMO-trypsinogen fusion in *N. benthamiana* leaves using plant viral vectors.
   A - Comassie-stained polyacryamide gels; B - Western blot with anti-trypsinogen antibodies (1:3000 dilution; 5 min exposure); C - testing for Trypsin enzymatic activity by using milk assay.
   dpi - days post-innoculation; M - molecular weight markers; U - uninfected tissue (control).

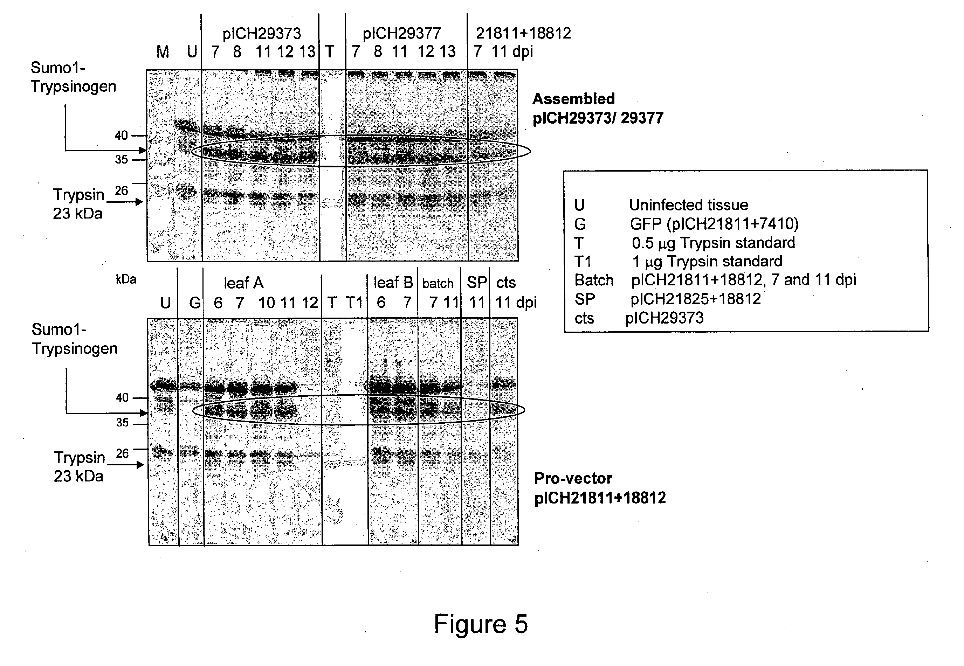

Fig. 5 shows expression of chloroplast-targeted recombinant SUMO-trypsinogen fusion in *N*. *benthamiana* leaves using plant viral vectors. Overnight cultures of agrobacteria for infiltration were 1:10 diluted. Tissue was harvested at between 6 and 13 dpi (days post infection), extracted with 6 volumes 1xLaemmli buffer and boiled before centrifugation. 0.05 ml/slot of supernatant was loaded onto the polyacrylamide gel.

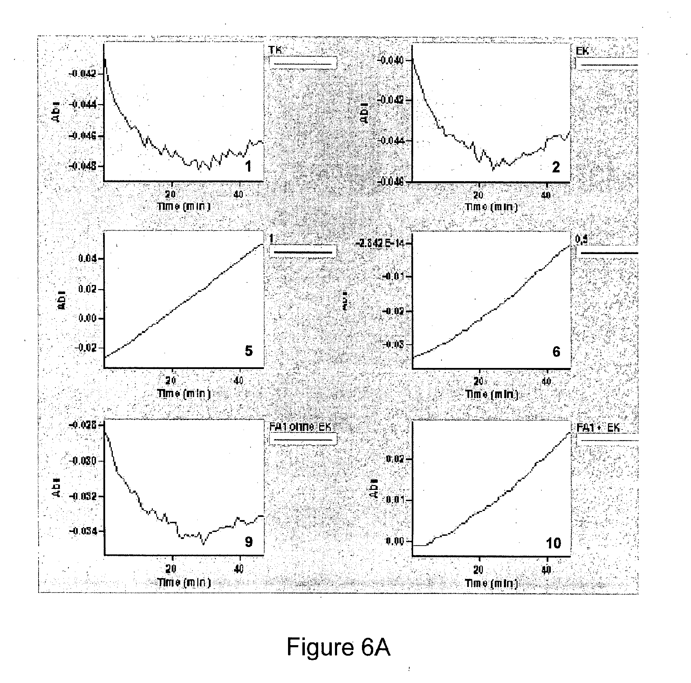

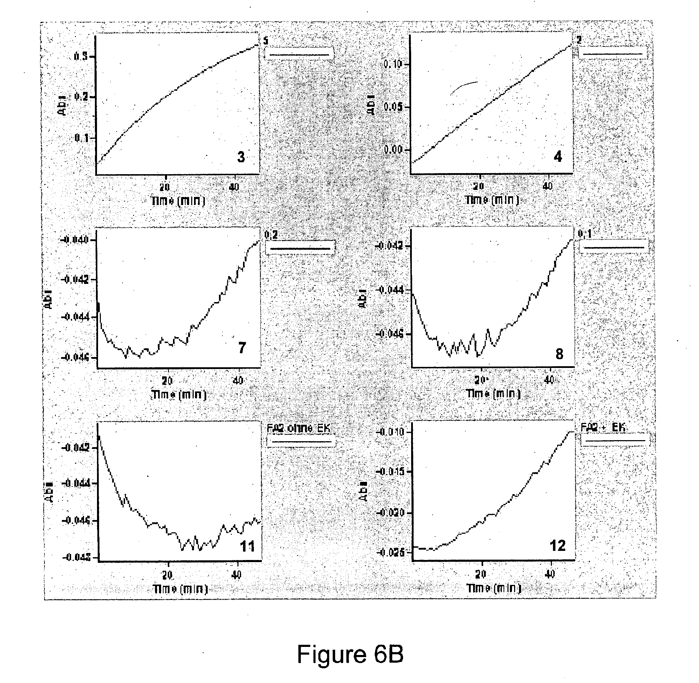

Fig. 6A and B show the kinetics of BAPNA cleavage with trypsin formed after trypsinogen processing with enterokinase. TK - trypsinogen control (graphic 1), EK - enterokinase control (graphic 2); Numbers 5; 2; 1; 0,5; 0,2 and 0,1 (graphics 3 to 8, respectively) are the respective concentrations (in µg/ml) of enterokinase cleaved trypsinogen standards; FA + EK - folding reaction with enterokinase (graphics 8 and 10); FA ohne EK - folding reaction without enterokinase (graphics 9 and 11.

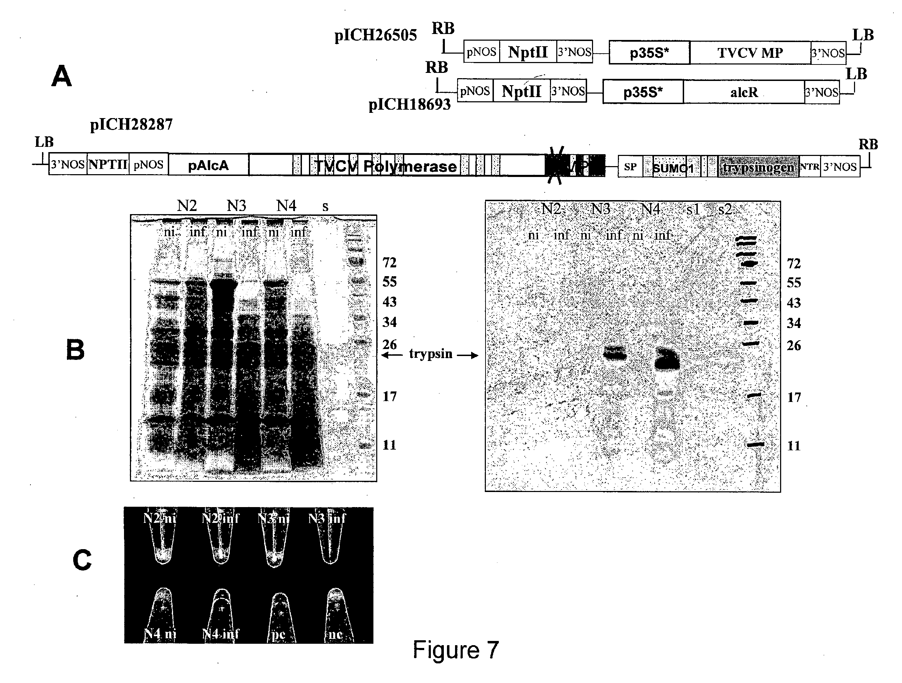

Fig. 7 shows expression of apoplast-targeted recombinant SUMO-trypsinogen fusion in stably transformed *N. benthamiana* plants carrying plant viral vector under control of inducible promoter. A - T-DNA regions of the binary vectors pICH26505, pICH18693 and pICH28287 TVCV polymerase - RNA-dependent RNA polymerase of Turnip Vein Clearing Virus with introns indicated by dotted portions; MP - viral movement protein with introns indicated by dotted portions; NTR - tobamoviral 3' non-translated region; 3' NOS - transcription termination region of nopaline synthase gene; SUMO1 - coding sequence with introns (indicated by dotted portions) of *Arabidopsis* SUMO1 gene; pNOS - promoter of nopaline synthase gene; pAlcA - inducible promoter of inducible *A. nidulans* alcA gene encoding alcohol dehydrogenase; alcR - transcriptional activator of the alc regulon of *Aspergillus nidulans*; p35S - 35S promoter of CaMV; NPTII - neomycin phosphotransferase II gene.
   B - comassie-stained polyacrylamide gel (left) and Western blot analysis (right) of total soluble protein extracted from transgenic plants transformed with pICH28287.
   N2, N3 and N4 - different transgenic *N. benthamiana* plants; ni - total soluble protein extracted from not induced (infiltrated) plant material; inf - total soluble protein extracted 7 days after infiltration of plants with pICH26505, pICH18693 and 2% ethanol; s, s1 and s2 - commercially available trypsin loaded at the concentrations 1.3 microgram, 50 nanogram and 100 nanogram, respectively.
   C - test for trypsin activity using digestion of milk proteins. nc - negative control (no trypsin was added); pc - positive control (1 µl of commercially available bovine trypsin from ICN Biomedicals, CA, USA, 1 mg/ml in 1 mM HCl).

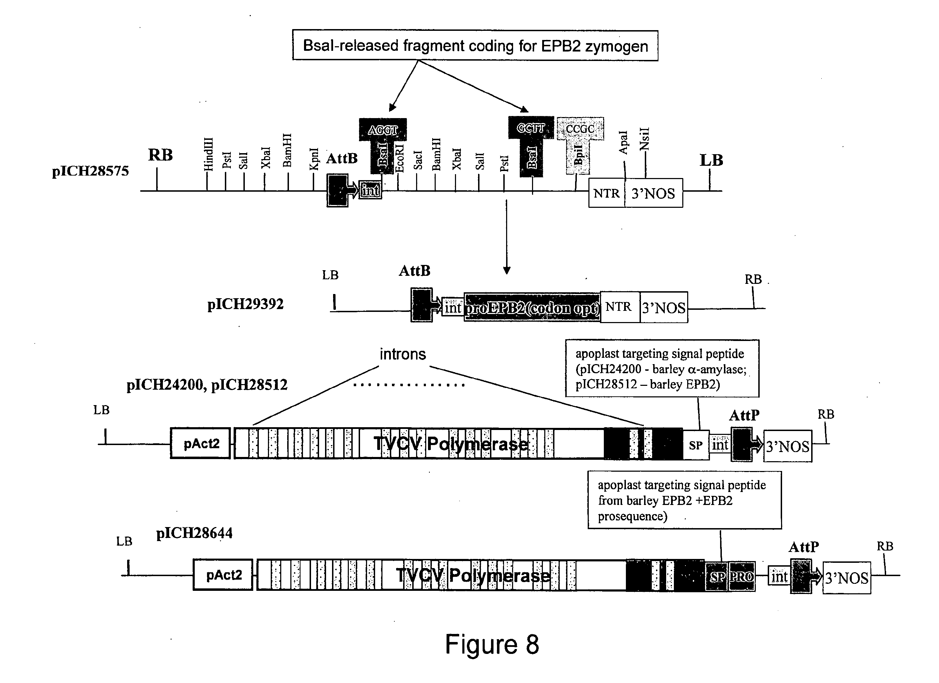

Fig. 8 depicts T-DNA regions of the binary vectors pICH28575, pICH29392, pICH24200, pICH28512 and pICH28644. Cloning strategy for codon-optimized EPB2 zymogen resulting in pICH29392 3' provector is shown in upper part of the figure. pAct2 - transcription promoter of Arabidopsis ACTIN2 gene; TVCV polymerase - RNA-dependent RNA polymerase of Turnip Vein Clearing Virus with introns indicated by dotted portions; MP - viral movement protein with introns indicated by dotted portions; NTR - viral 3' non-translated region; 3' NOS - transcription termination region of nopaline synthase gene; SUMO1 - coding sequence with introns of Arabidopsis SUMO1 gene; pNOS - promoter of nopaline synthase gene; AttP and AttB - recombination sites recognised by site-specific integrase phC31.

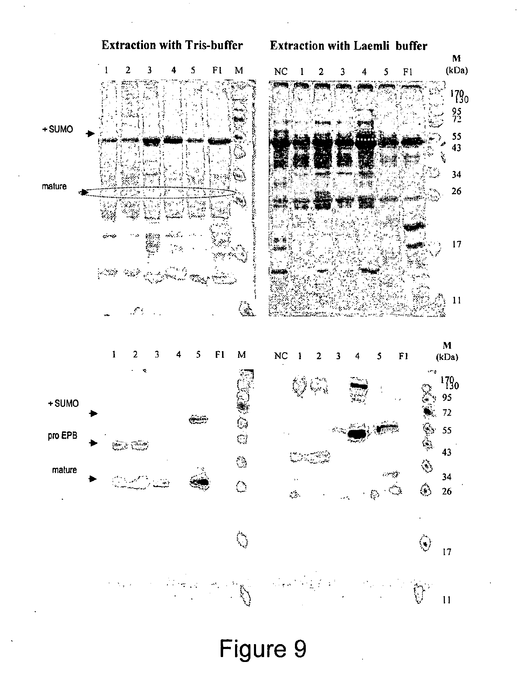

Fig. 9 shows shows the expression of apoplast- and chloroplast-targeted recombinant SUMO-EPB2 zymogen fusions in *N. benthamiana* leaves using plant viral vectors. Plant tissue was harvested 8 dpi (days post infection), extracted with 10 (w/v) volumes of 1xLaemmli buffer or 5 (w/v) of tris extraction buffer and boiled before centrifugation. The 0.05 ml/slot of supernatant was loaded onto the polyacrylamide gel. In case of tris extraction buffer supernatant before loading was diluted with equal volume of 2x Laemmli buffer. Expression of EPB2 (pICH29392) with: (1) barley apoplast targeting signal peptide (pICH24200); (2) its own (EPB2) apoplast targeting signal peptide(pICH28512); (3) EPB2 apoplast targeting signal peptide and prosequence (pICH28644); (4) chloroplast targeting transit peptide-SUMO fusion (pICH21811); (5) apoplast targeting signal peptide-SUMO fusion (pICH21825); F1 - *Yersinia pestis* F1, expressed with barley apoplast targeting signal peptide; NC - negative control (protein extracted from not infiltrated plant leaf material). The positions of mature EPB2 protein and SUMO1-EPB2 zymogen fusions on Comassie-stained gels are elipsed. Western blot analysis (lower panel) was performed with 1000x diluted anti-EPB2 antibodies.
Fig. 10 shows multiple alignments of sequences from different SUMO proteins. The program used: AlignX (Vector NTI Suite 7.1), based on the ClustalW algorithm.
   (A) - alignment of ten different SUMO protein sequences of animal, yeast and plant origin.
      *Mus musculus*: SMT3.2_MM - SUMO2 (Acc. No. NP_579932): SEQ ID NO: 17;
      *Mus musculus* SMT3.3_MM - SUMO3 (Acc. No. EDL31801): SEQ ID NO: 18;
      *Mus musculus* SMT3_MM - SUMO1 (Acc. No. NP_033486): SEQ ID NO: 19;
      *Oryza sativa*: SMT3_OS - SUMO1 (Acc. No. P55857)): SEQ ID NO: 20;
      *Saccharomyces cerevisiae*: SMT3_SC - SUMO1 (Acc. No. NP_010798): SEQ ID NO: 21;
      *Arabidopsis thaliana*: SUMO1_AT (Acc. No. P55852)): SEQ ID NO: 22;
      SUMO2_AT (Acc. No. NP_200327): SEQ ID NO: 23;
      SUMO3_AT (Acc. No. NP_200328): SEQ ID NO: 24;
      SUMO4_AT (Acc. No. NP_199683): SEQ ID NO: 25;
      SUMO5_AT (Acc. No. NP_565752): SEQ ID NO: 26;
      Identities - 4.1 %; positives - 61.8%. The ubiquitin domain is outlined.
   (B) Alignment of five different *Arabidopsis thaliana* SUMO protein sequences SEQ ID NOs: 22 to 26 from top to bottom: Identity - 13.9%, positives - 66.4%.

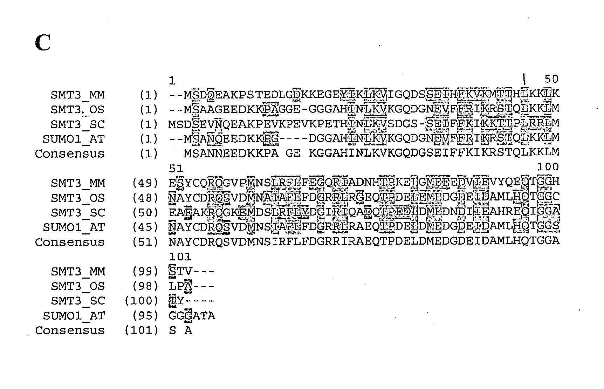

Fig. 11 Continuation of Fig. 10. Alignment of SUMO1 protein sequences derived from
   mouse (*Mus musculus):* SEQ ID NO: 19,
   rice (*Oryza sativa*): SEQ ID NO: 20 ;
   yeast *(Saccharomyces cerevisiae)*: SEQ ID NO: 21; and
   arabidopsis *(Arabidopsis thaliana*): SEQ ID NO: 22;
   Identity - 26.4%; positives - 92.5%.
Fig. 12 Map of pICH29090: sequence encoding for apoplast targeting signal peptide is shown in bold. The sequence of pICH29090 is shown in SEQ ID NO: 1. The sequence encoding the apoplast targeting signal peptide is shown in SEQ ID NO: 2.
Fig. 13 Map of pICH29373: the sequence of pICH29373 is identical to the one of pICH29090, except that the sequence encoding for apoplast targeting signal peptide of pICH29090 is replaced by the sequence encoding for artificial transit peptide shown in SEQ ID NO: 3.

### DETAILED DESCRIPTION OF THE INVENTION

It was previously shown that the expression level of recombinant proteins of interest can be improved via fusion with other proteins including SUMO (Butt et al., 2005, Protein Expr Purif., 43, 1-9; Marblestone et al., 2006, Protein Sci., 15, 182-189; Su et al., 2006, Protein Pept. Lett., 13, 785-792). Weeks et al., 2007, Protein Expr Purif., 53, 40-50; US2004018591; EP1654379). To the best of our knowledge, SUMO has so far not been used for improving expression levels of proteins of interest in plant expression systems, notably for cytotoxic proteins such as proteases.

The process of producing a protease of interest according to the invention involves expression of said protease of interest as a fusion protein which is compartmentalised within plant or plant cell by means of signal peptide or transit peptide-mediated targeting of the fusion protein. In the apoplast, the fusion protein may be processed to active protease, while in the chloroplast the fusion protein may be accumulated as protein inclusion bodies.

In the first step of the process of the invention, a plant or plant cells are transformed or transfected with a nucleotide sequence having a coding sequence encoding said fusion protein having a signal or transit peptide. Transformation may produce stably transformed plants or plant cells, e.g. transgenic plants. Alternatively, said plant or plant cells may be transfected for transient expression of said fusion protein. Several transformation or transfection methods for plants or plant cells are known in the art and include Agrobacterium-mediated transformation, particle bombardment, PEG-mediated protoplast transformation, viral infection etc.

Said nucleotide sequence may be DNA or RNA depending on the transformation or transfection method. In most cases, it will be DNA. In an important embodiment, however, transformation or transfection is performed using RNA virus-based vectors, in which case said nucleotide sequence is RNA.

Said nucleotide sequence comprises a coding region encoding a fusion protein. Said fusion protein comprises the SUMO protein. Said fusion protein further comprises a precursor of the protease of interest or zymogen that upon processing yields active protease (reffered to as "protease of interest" in the following). Said protease of interest may be any protease that can be produced and isolated according to the process of the invention. It may be produced in an unfolded, misfolded or in a natural, functional folding state. The latter possibility is preferred.

Said fusion protein further comprises a signal peptide functional for targeting said fusion protein to the apoplast or for targeting said fusion protein to plastid. The apoplast targeting may be achieved with a signal peptide that targets the fusion protein into the endoplasmatic reticulum (ER) and the secretory pathway. All signal peptides of proteins known to be secreted or targeted to the apoplast may be used for the purposes of the invention. Preferred examples are the signal peptides of tobacco calreticulin, barley or rice amylase. Signal peptides that target a protein to plastids are also referred to as "transit peptides". Any transit peptides can be utilized for practicing said invention. Preferred examples are artificial transit peptides or transit peptides of small subunit rubisco from tobacco. For functional targeting the transit peptide or the signal peptide is positioned at the N-terminus of the fusion protein.

After the fusion protein has been expressed by a plant, the protease of interest or a fusion protein comprising the protease of interest can be isolated from the plant or plant cell by standard protein purification methods. The isolation of a protease of interest or a fusion protein thereof can be facilitated by incorporating a purification tag into the protease or fusion protein. Such systems are commercially available e.g. from Amersham Pharmacia Biotech, Uppsala, Sweden. A specific example frequently used for removing a His-tag is the factor Xa system.

In another embodiment of said invention, an isolated fusion protein can be processed for releasing the protease of interest (e.g. trypsin) from said fusion by using enterokinase that specifically cleaves the zymogen (trypsinogen) at the N-terminus, whereby the active protease (e.g. trypsin) is released.

Construction of the nucleotide sequence of the invention may be done according to standard procedures of molecular biology. The nucleotide sequence may contain a plant-specific promoter operably linked to said coding sequence and a transcripiton terminator after said coding sequence. In the case of stable transgenic plants, inducible expression of the fusion protein may be achieved if desired by an appropriately selected inducible expression system. In a preferred embodiment, virus-based vectors under control of alcohol-inducible system are used for performing this invention. Construction of such viral vectors are described in the reference examples and in the numerous publications.

The nucleotide sequence comprising the coding sequence encoding the fusion protein of the compartmentalized with the help of signal or transit peptide may be delivered into the plant cell preferably using a DNA or an RNA vector. The recombinant protein fusion is expressed and then targeted to the intercellular space (apoplast) in case of fusion with signal peptide or to plastid. The plants with said fusion protein may then be subjected to processing. Dependent from the form in which the protease is accumulated in the plant, the downstream processing might incorporate protein fusion refolding and cleavage in order to produce active protease (in case of plastids compartmentalization), or lead directly to the isolation of active protease (in case of apolplast targeting).

Various methods can be used to deliver the nucleotide sequence of the invention using a vector into the plant cell, including direct introduction of said vector into a plant cell by means of microprojectile bombardment, electroporation or PEG-mediated treatment of protoplasts (for review see: Gelvin, S.B., 1998, Curr. Opin. Biotechnol., 9, 227-232; Hansen & Wright, 1999, Trends Plant Sci., 4, 226-231). Plant RNA and DNA viruses also present efficient delivery systems (Hayes et al., 1988, Nature, 334, 179-182; Palmer et al., 1999, Arch. Virol., 144, 1345-1360; Lindbo et al., 2001, Curr. Opin. Plant. Biol., 4, 181-185). Vectors can deliver a transgene either for stable integration into the genome of the plant (direct or Agrobacterium-mediated DNA integration) or for transient expression of the transgene ("agroinfiltration").

Different vectors may be used to express fusion protein in plant or plant cell. Suitable vectors for practicing said invention are the plant viral vectors. In one embodiment, RNA viral vectors are used. The use of such vectors for optimization of proteins expression and for large-scale production are described in detail in numerous publications (Marillonnet et al., 2004, Proc Natl Acad Sci U S A, 101:6852-6857; Marillonnet et al., 2005, Nat Biotechnol., 23:718-723; Giritch et al.,2006, Proc Natl Acad Sci U S A., 103, 14701-14706; Santi et al., 2006, Proc Natl Acad Sci U S A., 103, 861-866). In one embodiment, cloning of a bovine trypsinogen gene (Fig. 1) into 3' part of plant viral vector (3' provector) is described (see example 1, Fig. 2). Such 3' provector can be assembled into plant viral vector via site-specific recombination mediated by DNA recombinase (in said embodiment by phage C31 integrase). Using this approach, the 3' provector carrying the coding sequence of the invention can be fused in frame to any other coding sequence of interest. The approach allows to optimize the expression level of recombinant protein of interest in the most convenient and speedy way.

We have tested trypsinogen expression of many different fusions including translational fusions with five different *A. thaliana* SUMO proteins.The best results were obtained for SUMO1-trypsinoge fusion. Based on the results of studies with provectors, assembled plant viral vectors were designed for production of proteases in plant tissues. In Fig. 3 schematic representations of assembled viral vectors with trypsinogen targeted into apoplast (pICH29090) and chloroplast (pICH29373) are shown.

In yet another embodiment of this invention (example 2), the results of apoplast- and chloroplast-targeted trypsinogen exression using transient expression from provectors as well as from assembled viral vectors are described. It is evident from the results of protein (predominantly rubisco) degradation on comassie-stained gel and protease activity measurement using milk assay (Figure 4, A and C, respectively) that apoplast-targeting of trypsinogen leads predominantly to the formation of active trypsin. This finding is also confirmed by Western blot analysis with commercially available anti-trypsinogen antibodies (Fig. 4, B).

Targeting of trypsinogen into chloroplasts did not produce visible degradation of plant proteins, but resulted in major comassie-stained band on polyacryamide gel corresponding in size to a SUMO-trypsinogen fusion (Fig. 5). Accumulation of large amounts (ca. 2 mg/g of fresh leaf biomass) of said fusion in leaf chloroplasts is likely the result of formation of protein inclusion bodies, like in bacterial cells. Formation of inclusion bodies in chloroplasts is well known (Ketchner et al., 1995, Biol Chem., 270, 15299-15306; De Cosa et al., 2001, Nat Biotechnol., 19, 71-74; Fernández-San Millán et al., 2003, Plant Biotechnol J., 1, 71-79; Fernández-San Millán et al., 2007, J Biotechnol., 127, 593-604). The similarity to protein inclusion bodies from bacterial cells allows to use established technologies for solubilisation and refolding of bacterial protein inclusion bodies (for review and practical guide see: Singh SM, Panda AK. 2005, J Biosci Bioeng., 99, 303-310; Panda AK. 2003, Adv Biochem Eng Biotechnol., 85, 43-93; Cabrita LD, Bottomley SP. 2004, Biotechnol Annu Rev.;10, 31-50; Mukhopadhyay A. 1997, Adv Biochem Eng Biotechnol., 56, 61-109; Mayer M, Buchner J. 2004, Methods Mol Med.;94, 239-54; Misawa S, Kumagai I. 1999, Biopolymers., 51, 297-307). Indeed, in another embodiment of the invention (example 3), a successful approach for extraction and refolding of SUMO1-trypsinogen fusion is described by using slightly modified protocols for extraction of trypsinogen from bacterial inclusion bodies (Hohenblum et al., 2004, J. Biotechnol., 109, 3-11; Ahsan et al., 2005, Mol. Biotechnol., 30, 193-205; Király et al., 2006, Protein Expr. Purif., 48, 104-111). Products of folding reaction were treated with enterokinase and then tested for the formation of enzymatically active trypsin using the kinetics of BAPNA cleavage. The results of these experiments are shown in figure 6. It is evident from presented data that refolded and enterokinase-treated SUMO1-trypsinogen fusion produces active trypsin capable of digesting the substrate BAPNA (see graphics 10 and 12 of figure 6).

For large scale production of proteases in plants, a transgenic version of production host may be advantageous compared to transient expression system. Among transgenic vectors, those providing for controllable expression of the coding sequence of the invention are preferred. Controllable expression can help to further minimize cytotoxic effect of protease. In case of stable integration of a vector expressing a SUMO-trypsinogen fusion into a plant genome, controllable vectors based on inducible expression of said vector is preferred. In the present invention, inducible promoters can be used to trigger production of a protease of interest in plants or plant cells. Inducible promoters can be divided into two categories according to their induction conditions: those inducible by abiotic factors (temperature, light, chemical substances) and those that can be induced by biotic factors, for example, pathogen or pest attack. Examples of the first category include, but are not limited, heat-inducible (US 05187287) and cold-inducible (US05847102) promoters, a copper-inducible system (Mett et al., 1993, Proc. Natl. Acad. Sci., 90, 4567-4571), steroid-inducible systems (Aoyama & Chua, 1997, Plant J., 11, 605-612; McNellis et al., 1998, Plant J., 14, 247-257; US06063985), an ethanol-inducible system (Caddick et al., 1997, Nature Biotech., 16, 177-180; WO09321334; WO0109357; WO02064802), isopropyl beta-D-thiogalactopyranoside (IPTG)-inducible system (Wilde et al., 1992, EMBO J., 11:1251-1259) and a tetracycline-inducible system (Weinmann et al., 1994, Plant J., 5, 559-569). One of the latest developments in the area of chemically inducible systems for plants is a chimaeric promoter that can be switched on by glucocorticoid dexamethasone and switched off by tetracycline (Bohner et al., 1999, Plant J., 19, 87-95). Chemically inducible systems are the most suitable for practicing the present invention. For a review on chemically inducible systems see: Zuo & Chua, ( 2000, Current Opin. Biotechnol., 11, 146-151) and Moore et al., (2006, Plant J., 45: 651-683). It will be clear for the skilled person that any proteins required for the functionality of the chosen inducible system such as repressors or activators have to be expressed in said plant or said plant cells for rendering the inducible system functional. In one embodiment of the invention, ethanol inducible system for controlled release of viral replicon in plant cell is used. In example 4, an alcohol-inducible system described in detail in WO2007137788 was used for inducible expression of apoplast-targeted SUMO1-trypsinogen fusion. The results obtained demonstrate that tightly controlled inducible expression of protease from plant viral vetor is obtained. Figure 7 (B, C) shows expression of enzymatically active trypsin in different transgenic plants under inducibe conditions.

In example 5 of this invention, we present data of SUMO-EPB2 protease precursor expression in plant cells. It is evident (Figure 9, upper right panel, line 4) that a very high expression level of SUMO-EPB2 protease precursor fusion targeted into chloroplasts was achieved. Like in the case with chloroplast-targeted SUMO-trypsinogen fusion, SUMO-EPB2 precursor fusion accumulates in chloroplasts in the form of inclusion bodies that require strong denaturing buffers for their extraction from plant tissue. The extracted protein can be refolded in a way similar to the one described for chloroplast-targeted trypsinogen fusion with SUMO.

Considering that protease protein production in this invention includes the fusion of the protease with a signaling or transit peptide and SUMO, the precise separation of the protein of interest from the fusion protein shall be considered. In the invention, the use of SUMO and a protease precursor in such fusions introduces at least two cleavage sites. One cleavage site is located between the C-terminus of SUMO protein and the N-terminus of the protease precursor. This cleavage site is recognized by SUMO-specific proteases. Therefore, separation of the protease from SUMO is not an issue. Plant cells like all other eukaryotes, contain potent SUMO proteases that cleave proteins at the end of SUMO, thus precisely removing from SUMO any C-terminal extensions and fusions (Kurepa et al., 2003, J. Biol. Chem., 278, 6862-6872; Colby et al., 2006, Plant Physiol., 142, 318-332; Novatchkova et al., 2004, Planta, 220, 1-8; Hay, RT., 2004, Trends Cell Biol., 17, 370-376; Johnson et al., 2004, Annu Rev Biochem., 73, 355-382). In the invention, the protease precursor or zymogen is used for fusion with SUMO protein. The protease precursor contains yet another cleavage site not far from the N-terminus of said precursor. Cleavage at this cleavage of said site is important for the maturation of the precursor into the active protease. In case of trypsinogen, precursor of trypsin, enterokinase-mediated removal of a hexapeptide from the N-terminal end of trypsinogen produces trypsin. From our results with apoplast targeted SUMO-trypsinogen fusion, it is evident that a protease with enterokinase-like activity capable of a cleaving hexapeptide from trypsinogen must be present in plants.

The presence of a cleavage site within the zymogen allows removal of any fusion protein linked to the N-terminus of said zymogen. Therefore, the use of intact C-terminus of SUMO protein in order to provide cleavage with a SUMO-specific proteases is not necessary in the invention. This allows to use a truncated version of SUMO protein in the fusion protein of the invention and other derivatives of SUMO protein.

### EXAMPLES

### EXAMPLE 1

### Cloning of bovine coding sequences encoding for trypsinogen and trypsin and their integration into plant viral vectors

Commercially available calf thymus genomic DNA (Sigma-Aldrich., cat. No. D4764) was used as the template for cloning coding regions for trypsinogen and trypsin proteins. The partial coding sequence for activation peptide and pancreas cationic trypsinogen (Core Nucleotide database Acc. No D38507, also see Fig. 1 (A) was used for primers design. Nine primers containing Bsal restriction sites were synthesized in order to amplify exon sequences encoding bovine cationic trypsin and trypsinogen proteins. The primer sequences and general scheme of cloning strategy are shown in Figure 2. The A-tailed PCR products were subcloned into pGemT T-tailed vector (Promega, Cloning kit Cat. No. A3600). Nine primers were designed in order to amplify the coding sequences from genomic DNA and avoid introns. The primers also introduced flanking Bsal sites into PCR fragments. Five different fragments covering cDNA coding region for trrypsinogen/trypsin were independently subcloned in pGemT vectors and sequenced. Clones with correct sequences were used for assembly of coding sequences for trypsinogen or trypsin proteins by subcloning appropriate PCR clones as Bsal fragments into Bsal and HindIII-digested vector pICH10990 yielding vectors pICH18812 (trypsinogen, Fig 2) and pICH18820 (trypsin, Fig.2). Use of Bsal restriction sites provides the universal approach to create any desired compatible sticky ends flanking digested DNA fragments and thus allows to perform correct assembly of several DNA fragments in one cloning step. The vectors pICH18812 and pICH18820 were further used as 3' provectors in site-specific recombination-mediated assembly of DNA encoding for complete viral vector. The principle of such DNA modules assembly *in planta* is described by Marillonnet et al., 2004, Proc. Natl. Acad. Sci. USA, 101, 6852-6857. The approach allows high throughput testing of different targeting signals and fusion proteins in combination with the protein of interest for optimazing said protein expression level.

The *Arabidopsis thaliana* SUMO1 gene (gene ID 828791) was cloned using genomic DNA as template for PCR amplification. The PCR product containing two original SUMO1 introns, was cloned into intermediate vectors using standard molecular biology techniques ((Sambrook, Fritsch & Maniatis, 1989, Molecular Cloning: a Laboratory Manual, CSH, NY), and used in constructs design.

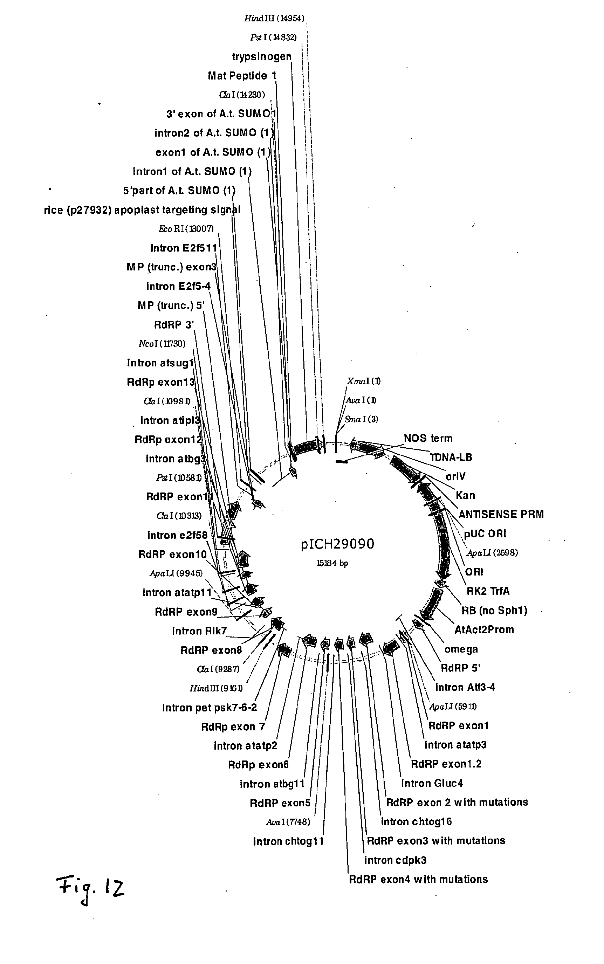

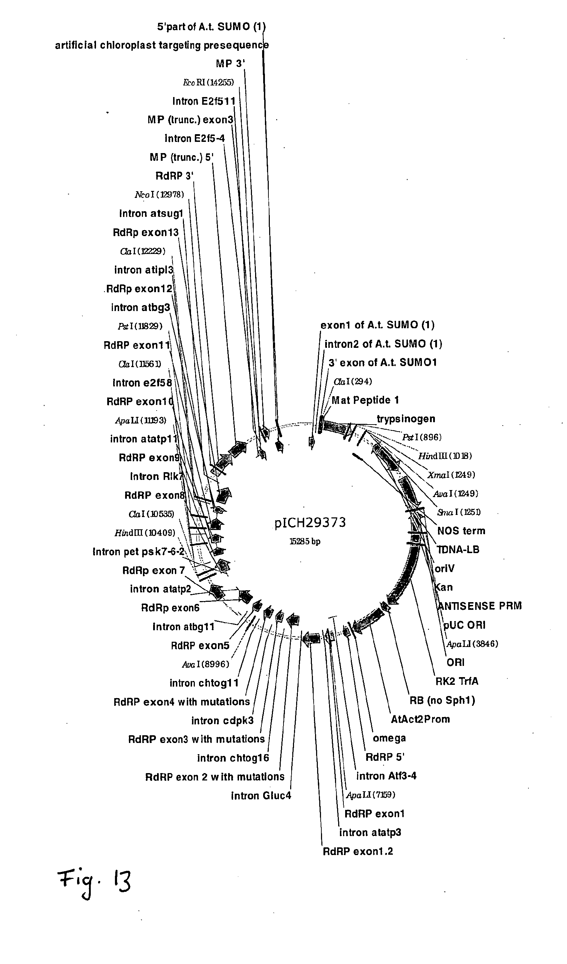

The viral vector modules with signal peptide, transit peptide-SUMO1 and signal peptide-SUMO1 fusions encoding for 5'-viral pro-vectors and assembled viral vectors were designed using cloning approaches, as it described by Marillonnet et al., 2005, Nat. Biotechnol., 23, 718-723. The restriction maps of whole plasmids and complete coding sequences for the T-DNA regions of assembled viral vectors pICH29090 and pICH29373 (Fig. 3) are shown figures 12 and 13, respectively.

The complete sequence of pICH29090 is shown in SEQ ID NO: 1. The sequence encoding the apoplast targeting signal peptide of pICH29090 is shown in SEQ ID NO: 2. The sequence encoding the apoplast targeting signal peptide of pICH29373 is shown in SEQ ID NO: 3. The sequence of pICH29373 is identical to that of pICH29090 except that the sequence encoding the apoplast targeting peptide of pICH29090 is replaced by the sequence encoding the plastid transit peptide shown in SEQ ID NO: 3.

### EXAMPLE 2

### Transient expression of bovine trypsinogen in N. benthamiana using plant viral vectors Agroinfiltration

All constructs described in example 1 were electroporated into Agrobacterium tumefaciens GV3101. Agroinfiltrations of *N. benthamiana* plants were done essentially as described in Marillonnet et al., 2004, Proc Natl Acad Sci USA, 101:6852-6857. In case of provectors use three agrobacterial strains containing 5' provector encoding for targeting signal peptide and SUMO1 fusion or any othe fusion/targeting sequence (not shown), 3' provector encoding the trypsinogen or trypsin genes and a source of a site-specific recombinase (pICH14011, Fig. 3) for assembly of viral pro-vectors *in planta* via site-specific recombination into viral vector were mixed together and used for infiltration. Small-scale infiltrations were done with a syringe; large-scale infiltrations were done using a vacuum device (Marillonnet et al., 2005, Nat Biotechnol., 23:718-723). Agrobacterial strains containing assembled viral vectors (pICH29090, pICH29373, Fig. 3) were agro-infiltrated independently.

### Analysis of SUMO1 - trypsinogen fusion expressed in N. benthamiana leaves

All recombinant protein fusions were extracted from infiltrated *N. benthamiana* leaves 7-12 days after infiltration and analysed by electrophoretic separation in polyacrylamide gels as previously described (Marillonnet et al., 2004, Proc Natl Acad Sci U S A, 101:6852-6857; Marillonnet et al., 2005, Nat Biotechnol., 23:718-723). Plant leaf tissue was harvested from different leaves of young (y) or old plants. Tissue was extracted with 3 volumes of 0.15 M Tris-HCl pH8.1; 2mM EDTA, incubated for 10 minutes on ice and centrifuged for 12 minutes at 13 Krpm, 4 °C. Supernatant was mixed with equal volume of 2x Laemmli buffer (125 mM Tris/HCl pH 6.8, 10 % mercaptoethanol, 20 % glycerol, 0.01 % bromphenole blue, 4 % SDS) and 0.004 ml of mixture corresponding to 1.3 mg of starting leaf tissue was loaded on gels.

The results of electrophoretic analysis are shown in Figure 4 (A). The position of trypsin bands was identified by using Western blotting (Fig.4-B) with anti-bovine trypsinogen polyclonal rabbit antibodies (Rockland/Biomol GmbH. Hamburg, cat No. 100-4180). It corresponded to clearly visible 23 KDa comassie-stained band on polyacrilamyde gel.

### Measurement of plant-made recombinant trypsin activity

The relative activity of plant-expressed trypsin in comparison with commercially available trypsin samples (ICN Biomedicals Inc., cat. 101789, 10 mg/ml in 1 mM HCl, working solution 1:10 to 1:100 diluted in 1 mM HCL) was measured by using milk assay. The assay was performed as follows:
0.005-0.02 ml of plant extract in 0.15 M Tris-HCl pH8.1; 2mM EDTA 8,0 buffer is mixed with 0.02 ml of 3% solution of dry milk powder (Roth, Cat. No. T145.2) in TBS (25 mM tris-HI, 142 mM NaCl) and incubated at room temperature until positive control sample with commercially available trypsin does not start to clarify the milk solution due to the digestion of milk protein. Clear solution in test samples means the presence of active trypsin. If the solution remains milky, no trypsin activity is present. The results of the test are shown in Fig. 4-C.

### Analysis of chloroplast-targeted SUMO1-trypsinogen fusion

Agroinfiltration of plants with assembled viral vector and provectors providing for expression of chloroplast-targeted SUMO1-trypsinogen fusion was performed as described above for apoplast-targeted SUMO1-trypsinogen fusion. The results of electrophoretic analysis of the expression level are shown in Fig. 5 (comassie-stained bands corresponding to recombinant protein fusion are circled). It is evident that a large amount (ca. 2 mg/g of fresh leaf biomass) of inactive (no degradation of protein in comassie-stained gel was detected) SUMO1-trypsinogen fusion accumulated in plant tissue.

### EXAMPLE 3

### Extraction and reactivation of chloroplast targeted SUMO1-trypsinogen fusion

### Extraction of SUMO1-trypsinogen fusion from chloroplasts

The protein fusion accumulated in chloroplasts can be extracted with SDS-PAGE sample (Laemmli) buffer at 95°C (see example 2) and at least partially with buffers containing chaotropes. The current extraction method was established based on methods suitable for solubilisation of inclusion bodies (IBs) occurring in many recombinant protein expressions in *E. coli* including trypsinogen (Buswell et al., 2002, Biotechnol. Bioeng., 77, 435-444; Hohenblum et al., 2004, J. Biotechnol., 109, 3-11; Ahsan et al., 2005, Mol. Biotechnol., 30, 193-205; Király et al., 2006, Protein Expr. Purif., 48, 104-111). 5.5 g of plant leaf material containing chloroplast targeted SUMO1-trypsinogen fusion was sequentially treated with the set of following extraction buffers:
E1: 100 mM Tris-HCl, 200 mM NaCl, 1 mM EDTA, pH 8.5
E2: 60 mM EDTA, 2% Triton-X100, 1,5 M NaCl, pH 8.8
E3: 4 M urea, acetic acid, pH 4.0
E4: 6 M GuaHCl, 100 mM Tris-HCl, 1 mM EDTA, 100 mM DTT, pH 8.8

*Extraction 1:* 30 ml buffer E1 was added to the leaf material. The suspension was thoroughly mixed using an ultraturrax (6-7 times 30 s), and kept on ice between the 3 mixing steps. Overall mixing time 10 min. The suspension was centrifuged 15 min at 40000g and 4°C.

*Extraction 2*: 25 ml buffer E2 were added to the pellet of extraction step 1. The suspension was thoroughly mixed using an ultraturrax (3 times 1 min), and kept on ice between the mixing steps. Overall mixing time 10 min. The suspension was centrifuged 15 min at 40000g and 4°C.

*Extraction 3*: 25 ml buffer E3 were added to the pellet of extraction step 2. The suspension was thoroughly mixed using an ultraturrax (3 times 1 min) and kept on ice between the mixing steps. Overall mixing time 10 min. The suspension was centrifuged 15 min at 40000g and 4°C. This washing step was repeated 3 times.

*Extraction 4:* 10 ml buffer E4 was added to the pellet of extraction step 3. The suspension was thoroughly mixed using an ultraturrax (5 min), and incubated further for 1 h on a rolling mixer at room temperature. The suspension was centrifuged 15 min at 40000g and 4°C. The supernatant, assumed to contain solublised SUMO-trypsinogen, was applied to a HiPrep Desalting column to exchange the buffer to 8 M urea, acetic acid, pH 4.0. The fractions containing protein were pooled. All fractions were analysed by SDS-PAGE and Westernblot (not shown).

The final extraction of unsoluble proteins is achieved with the GuaHCl containing buffer. In preliminary experiments, this buffer was found to be suitable to extract more SUMO1-trypsinogen compared to a similar buffer containing 9 M urea as chaotrope.

Additional purification of extracted SUMO1-trypsinogen was carried out using Q Hyper D 20 anion exchange chromatography (Pall, Biosepra, code no. 200683, column 4.6x100mm). The sample was prepared by adding 0.5 volume of 200 mM Tris-HCl, 1 mM EDTA, pH8.5, to one volume of solubilised SUMO1-trypsinogen from extraction 4. The pH was adjusted to >8.0 by addition of 1 M NaOH and final solution was loaded onto the column. The elution was carried out by linear NaCl gradient (starting buffer: 50 mM Tris-HCl, 8 M urea, pH 8.5; final buffer: 50 mM Tris-HCl, 8 M urea, 1 M NaCl, pH 8.5; flow rate: 1ml/min; 20 column volumes, elution fraction volume: 0.5 ml.
8 M urea was used to keep SUMO-trypsinogen fusion solubilised. The resulting purity of SUMO-trypsinogen was comparable to typical proteins solubilised from bacterial inclusion bodies. The final pool contained an overall amount of 0.6 mg in 3 ml.

### SUMO1-trypsinogen folding and cleavage of fusion to produce active trypsin

The folding of purified SUMO1-trypsinogen fusion was carried out as described by Hohenblum and colleagues (J. Biotechnol., 109 (2004) 3-11) with some modifications. Concentration of the purified extract of SUMO1-trypsinogen to 150 µl was carried out by using Vivaspin 500 (Sartorius, MWCO 3,000; prod no. VS0191; Lot no. 07VS50030). Recovery from the Vivaspin concentrator included rinsing with 8 M urea, 50 mM Tris, pH 8.6. Final volume of the concentrated samples 700 µl. To the concentrated sample, 1 M DTE was added to a final concentration of 10 mM and the solution was incubated for 2.5 hours at 37°C. Then, 1 volume of 200 mM GSSG (oxidised glutathion), 8 M urea, pH 8.6 was added and the mixture was incubated for 3 hours at 37°C. After incubation, the buffer was replaced to 50 mM Tris-HCl, 8 M urea, pH 8.6 by using HiTrap desalting columns (GE Healthcare, cat. no. 17-1408-01). Then dilution of the solubilisates into folding buffer (two different buffers, 1:20 dilution; folding buffer 1: 50 mM Tris-HCl, 50 mM CaCl2, 3 mM GSH, 0.3 mM GSSG, pH 8.6; folding buffer 2: 50 mM Tris-HCl, 50 mM CaCl2, 700 mM Arg, 3 mM GSH) and incubation of the folding reaction was carried out at 4°C for at least 16 hours. After incubation, the folding reaction was concentrated 10-fold with Vivaspin 20 concentrators (Sartorius, MWCO 5,000; prod. no. VS2011, Lot 06VS2050). Then, the folding buffer was replaced by cleavage buffer (20 mM Tris-HCl, 50 mM NaCl, 2 mM CaCl2, pH 8.0) using HiTrap desalting column. The resulting samples were analysed by SDS-PAGE and activity assays were made (results are not shown).

In order to test the quality of SUMO1-trypsinogen folding, enterokinase (stock solution: 1 mg/ml of enterokinase (Sigma, cat. no. E0885) in cleavage buffer) was added to the folding samples. In case of a successful folding, enterokinase cleavage should lead to the formation of active trypsin that can be detected due to the cleavage of the chromogenic substrate BAPNA (Sigma, cat. no. B4875). The enterokinase cleavage and the analytical methods were established with commercially available trypsinogen. In accordance with the SUMO1-trypsinogen concentration in the folding reaction, the analytical method was established with trypsinogen concentrations between 0.1 and 50 µg/ml. The BAPNA assay system was optimised to detect very small amounts of trypsin in enterokinase treated folding reactions. The BAPNA assay was performed as follows:
Ten microliters of enterokinase were added to 990 µl folding sample in cleavage buffer and the mixture was incubated at 37°C for >16hours. The sample was pipetted into a cuvette and 50 µl of 2 mM BAPNA solution in cleavage buffer were added. The BAPNA cleavage was detected at 37°C by absorption spectroscopy at 405 nm over a time period of 60 min. The results (kinetic of absobtion ) were evaluated in comparison with results obtained from control samples obtained from trypsinogen standard. The results of cleavage experiments are shown in Figure 6.

### EXAMPLE 4

### Use of alcohol-inducible system for the expression of apoplast targeted SUMO-trypsinogen fusion in transgenic N. benthamiana plants

### Constructs design

The constructs for inducible expression of trypsin in transgenic plants are shown in Fig. 7A. Plasmid pICH28287 is very similar to the plasmid coding for assembled viral vector pICH29090 (Fig. 3) except that the promoter of Arabidopsis actin 2 gene was replaced with ethanol inducible alcA promoter and a frameshift mutation was introduced into the coding sequence of MP. Description of the ethanol-inducible system for expression of recombinant proteins in plants using standard transcriptional vectors was provided in detail in several publications (Caddick et al., 1997, Nature Biotech., 16, 177-180; WO09321334; WO0109357; WO02064802). Ethanol-inducible system used in this invention for controlling plant viral vector-based expression was described in detail in our PCT application WO 2007/137788.

*N. benthamiana* plants were transformed with pICH28287 according to standard protocols (Horsh et al., 1985, Science, 227, 1229-1231). Regenerated plants were analysed for the presence of the transgene by agroinfiltration with the constructs providing for alcR transcriptional activator and functional MP (pICH18693 and pICH26505, respectively, see Fig. 7-A) followed by ethanol treatment. Analysis of three (N2, N3, N4) such transgenic plants for the presence of trypsin enzymatic activity is shown in Fig. 7-B, C. Preparation of samples, gel-electorphoresis, Western blotting and milk assay were performed as described in example 2. Clearly, two out of three plants express trypsinogen upon induction. Activity is shown both in the milk assay and by degradation of proteins in the Coomassie-stained gel.

### EXAMPLE 5

### Cloning of barley (Hordeum vulgare) coding sequences encoding for cysteine endoprotease B (EPB2) precursor and its integration into plant viral vectors

The codon-optimised sequence of barley cysteine endoprotease B isoform 2 (EPB2) gene (GeneBank Acc. No. U19384) was custom-synthesized (GENEART AG, Regensburg, Germany). The sequence of the gene and its translation product are shown in Figure 1 (B). As a matter of convenience, the sequence was flanked with two Bsal sites that were used for recloning of the gene into Bsal digested provector pICH28575, yielding provector pICH29392. Schematic representations of provectors and cloning procedures are shown in Figure 8. The vector pICH29392 was further used as 3' provectors in site-specific recombination-mediated assembly of DNA encoding for complete viral vector. The principle of the assembly of such DNA modules *in planta* is described by Marillonnet et al., 2004, Proc. Natl. Acad. Sci. USA, 101, 6852-6857. This approach allows high-throughput testing of different targeting signals and fusion proteins in combination with the protein of interest for optimizing the expression level of said protein. The 5' provectors used in combination with pICH29392 are pICH24200, pICH28512, pICH28644 (Fig. 8) and pICH21811, pICH21825 (Fig. 3).

### Analysis of SUMO1- EPB2 zymogen fusion expressed in N. benthamiana leaves

All recombinant protein fusions were extracted from infiltrated *N. benthamiana* leaves 7-12 days after infiltration and analysed by electrophoretic separation in polyacrylamide gels as previously described (Marillonnet et al., 2004, Proc Natl Acad Sci USA, 101:6852-6857; Marillonnet et al., 2005, Nat Biotechnol., 23:718-723). It was found that the expression level reached a maximum 8 days after infiltration.

Plant leaf tissue was extracted with 5 volumes of tris extraction buffer (0.1 M Tris-HCl pH8.0; 5 mM EDTA, 2 mM mercaptoethanol, 0.1% SDS, 15% glycerol), incubated for 10 minutes on ice and centrifuged for 12 minutes at 13 Krpm, 4 °C. The supernatant was mixed with equal volume of 2x Laemmli buffer (125 mM Tris/HCl pH 6.8, 10 % mercaptoethanol, 20 % glycerol, 0.01 % Bromphenole blue, 4 % SDS) and incubated in a boiling water bath before loading on gels. Alternatively, plant leaf tissue was extracted with 10 volumes (w/v) of 1x Laemmli buffer (62.5 mM Tris/HCl pH 6.8, 5% mercaptoethanol, 10 % glycerol, 0.005 % Bromphenole blue, 2 % SDS). Extracts were incubated in a boiling water bath before loading on gels.

The results of electrophoretic analysis are shown in Figure 9. The position of mature EPB2 (25 kDa), EPB2 propeptide (38 kDa) and SUMO1-EPB2 propeptide fusion are shown by arrows. The positions of EPB2-containing bands was confirmed by using Western blotting (Fig. 9, lower panel) with anti-EPB2 polyclonal rabbit antibodies.

## Claims

1. A process of producing a protease in a plant or in plant cells, comprising
(a) providing a plant comprising a heterologous nucleotide sequence comprising a coding sequence encoding a fusion protein, said fusion protein comprising:
(i) an apoplast or plastid signal peptide;
(ii) a SUMO protein or a derivative of a SUMO protein; and
(iii) a zymogen of said protease, and
(b) expressing said fusion protein.

2. The process according to claim 1, wherein said plant or said plant cells provided in step (a) is/are stably transformed on a nuclear chromosome with said nucleotide sequence comprising said coding sequence.

3. The process according to any one of claims 1 or 2, wherein said nucleotide sequence encodes a replicon comprising said coding sequence or a transcript of said coding sequence.

4. The process according to claim 3, wherein said replicon is an RNA viral replicon.

5. The process according to any one of claims 1 to 4, wherein said nucleotide sequence comprises a promoter upstream of said coding sequence, said promoter allowing expression of said coding sequence in vegetative tissue of said plant.

6. The process according to claim 5, wherein said promoter is an inducible promoter.

7. A process of producing a protease in a plant or in plant cells, comprising providing a plant with a replicon comprising a coding sequence encoding a fusion protein, said fusion protein comprising:
(i) an apoplast or plastid signal peptide;
(ii) a SUMO protein or a derivative of a SUMO protein; and
(iii) a zymogen of said protease.

8. The process according to claim 7, where said replicon is a plant viral expression vector.

9. The process according to any one of claims 7 or 8, wherein said replicon is an RNA replicon, and said plant is provided with said RNA replicon by transforming said plant or said plant cells with a DNA vector encoding said RNA replicon or with two or more DNA vectors encoding together said RNA replicon.

10. The process according to any one of claims 1 to 9, wherein said derivative of a SUMO protein is capable of increasing the expression level of said protease or of a protein comprising said protease compared to the absence of said derivative.

11. The process according to any one of claims 1 to 10, wherein said derivative of a SUMO protein comprises an amino acid sequence segment of at least 50 contiguous amino acid residues, said segment comprising any one of the following amino acid consensus sequences:
- L/F/M-X₁₉-F/I-X₃-G/D-X₇-T/S-P/A-;
- L/F/M-X₁₉-F/I-X₃-G/D-X₇-T-P-;
- L-(X)₁₉-F-X₃-G-X₇-T-P-;
- L-X₁₉-F-X₃-G-X₇-T-P-X₁₈-G-G-;
- L/I-X-V/L-Xₐ-L-X₁₉-F-X₃-G-X₇-T-P-;
- L-X-K/R-X₁₇-F-X₃-G-X₇-T-P-;
- L-X-K/R-L/M-X₁₆-F-X₃-G-X₇-T-P-;
- L-X-K/R-UM-M-X₁₅-F-X₃-G-X₇-T-P-;
- L-X-K/R-L/M-M-X₁₅-F-X₃-G-X₇-T-P-X₁₈-G-G-;
- L-X-K/R-UM-M-X₁₅-F-X₃-G-X₇-T-P-X₂-L-;
- L-X-K/R-UM-M-X₁₅-F-X₃-G-X₇-T-P-X₂-L-D/E-X₂-D/E-;
- L-X-K/R-L/M-M-X₁₅-F-X₃-G-X₇-T-P-X₂-L-D/E-X₂-D/E-X₃-I-D/E-;
- L-X-K/R-L/M-M-X₁₅-F-X₃-G-X₇-T-P-X₂-L-D/E-X₂-D/E-X₃-I-D/E-X₆-G-G-;
- L/I-X-V/L-Xₐ-L-X-K/R-L/M-M-X₁₅-F-X₃-G-X₇-T-P-X₂-L-D/E-X₂-D/E-X₃-I-D/E-X₆-G-G-;
- L-K-V-K-X_{b}-L-X₁₉-F-X₃-G-X₇-T-P-;
- L-K-V-K-X_{b}-L-X-K-X-M-X₁₅-F-X₃-G-X₇-T-P-;
- L-K-V-K-X_{b}-L-X-K-X-M-X₂-Y-X₁₂-F-X₃-G-X₇-T-P-;
- L-K-V-K-X_{b}-L-X-K-X-M-X₂-Y-X₁₂-F-X₃-G-X₇-T-P-X₂-L-;
- L-K-V-K-X_{b}-L-X-K-X-M-X₂-Y-X₁₂-F-X₃-G-X₇-T-P-X₂-L-X₁₅-G-G-;
- L-K-V-K-X_{b}-L-X-K-X-M-X₂-Y-X₁₂-F-X₃-G-X₇-T-P-X₂-L-X₇-I-;
- L-K-V-K-X_{b}-L-X-K-X-M-X₂-Y-X₁₂-F-X₃-G-X₇-T-P-X₂-L-X₅-X-I-;
- L-K-V-K-X_{b}-L-X-K-X-M-X₂-Y-X₁₂-F-X₃-G-X₇-T-P-X₂-L-X₇-I-X₇-G-G-;
- L-K-V-K-X-Q-X_{c}-L-X-K-X-M-X₂-Y-X₁₂-F-X₃-G-X₇-T-P-X₂-L-X₇-I-X₇-G-G-;
- L-K-V-K-X_{b}-L-L/K-K-L/M-M-X₂-Y-X₁₂-F-X₃-G-X₇-T-P-X₂-L-X₇-I-X₇-G-G-;
- L-K-V-K-X-Q-X_{c}-L-L/K-K-L/M-M-X₂-Y-X₁₂-F-X₃-G-X₇-T-P-X₂-L-X₇-I-X₇-G-G-;
- L-X-K/R-L-X₁₆-F-X₃-G-X₇-T-P-;
- L-X-K/R-L-M-X₁₅-F-X₃-G-X₇-T-P-;
- L-X-K/R-L-M-X₅-R/K-Q/R-X₈-F-X₃-G-X₇-T-P-;
- L-X-K/R-L-M-X₅-R-Q-X₈-F-X₃-G-X₇-T-P-;
- L-X-K/R-L-M-X₅-R-Q-X₈-F-X₃-G-X₇-T-P-X₂-L-X-M-;
- L-X-K/R-L-M-X₅-R-Q-X₃-M-X₄-F-L-X₂-G-X₇-T-P-X₂-L-X-M-;
- L-X-K/R-L-M-X₅-R-Q-X₈-F-L-X₂-G-X₇-T-P-X₂-L-X-M-E-X₄-I-X₇-G-G-:
- L-X-K/R-L-M-X₅-R-Q-X₈-F-L-X₂-G-X-R-X₅-T-P-X₂-L-X-M-E-X₄-I-X₇-G-G-:
wherein
the amino acid consensus sequences are given in N-terminal to C-terminal direction; a is an integer of 17 or 18;
b is an integer of 16 or 17;
c is an integer of 14 or 15;
each letter stands for an amino acid residue;
X stands for any amino acid residue;
letters other than X stand for amino acid residues in the standard one-letter code;
a numerical subscript to a letter indicates that the amino acid residue defined by said letter is present contiguously and connected by peptide bonds as many times as indicated by the numerical value of the subscript;
"-" stands for a peptide bond connecting adjacent amino acid residues; and
"/" indicates that the amino acid position defined by two consecutive "-" can be occupied by any of the amino acid residues defined by letters separated by "/".

12. The process according to any one of claims 1 to 11, wherein said protease or a polypeptide comprising said protease is isolated from vegetative tissue of said plant.

13. The process according to any one of claims 1 to 12, wherein said plant or said plant cells belong to genus *Nicotiana*.

14. The process according to any one of claims 1 to 13, wherein said coding sequence contains one or more introns, notably in a region coding for said SUMO protein or said derivative of a SUMO protein.

15. The process according to any one of claims 1 to 14, wherein said fusion protein comprises an affinity tag for purifying said fusion protein or a fragment thereof by affinity purification.

16. The process according to any one of claims 1 to 15, further comprising
(c) isolating and purifying said protease or said zymogen or a fusion protein comprising said protease from vegetative tissue of said plant or from said plant cells, optionally followed by
(d) generating said protease from said zymogen or from said fusion protein by proteolytic clearvage.

17. The process according to any one of claims 1 to 16, wherein said protease is selected from trypsin and chymotrypsin.

18. Vector or nucleotide sequence comprising a coding sequence encoding a fusion protein, said fusion protein comprising an apoplast or plastid signal peptide, a SUMO protein or derivative of a SUMO protein, and a zymogen of said protease.

19. A transgenic plant or plant cells for expressing a protease or a fusion protein comprising said protease, said plant comprising a nucleotide sequence containing a promoter active in leaf tissue and, downstream of said promoter and operably linked thereto, a coding sequence encoding a fusion protein comprising a plastid or apoplast signal peptide, a SUMO protein or a derivative thereof, and a zymogen of said protease.


SEQUENCE LISTING

<110>  Icon Gentics GmbH

<120>  Method of Protease Production in Plants

<130>  EAD-14704

<160>  26

<170>  PatentIn version 3.1

<210>  1
<211>  15184
<212>  DNA
<213>  Artificial Sequence

<220>
<223>  expression vector

<400>  1
ccgggtaggg gcccagcggc cgctctagct agagtcaagc agatcgttca aacatttggc      60

aataaagttt cttaagattg aatcctgttg ccggtcttgc gatgattatc atataatttc     120

tgttgaatta cgttaagcat gtaataatta acatgtaatg catgacgtta tttatgagat     180

gggtttttat gattagagtc ccgcaattat acatttaata cgcgatagaa aacaaaatat     240

agcgcgcaaa ctaggataaa ttatcgcgcg cggtgtcatc tatgttacta gatcgacctg     300

catccacccc agtacattaa aaacgtccgc aatgtgttat taagttgtct aagcgtcaat     360

ttgtttacac cacaatatat cctgccacca gccagccaac agctccccga ccggcagctc     420

ggcacaaaat caccactcga tacaggcagc ccatcagtca gatcaggatc tcctttgcga     480

cgctcaccgg gctggttgcc ctcgccgctg ggctggcggc cgtctatggc cctgcaaacg     540

cgccagaaac gccgtcgaag ccgtgtgcga gacaccgcgg ccgccggcgt tgtggatacc     600

tcgcggaaaa cttggccctc actgacagat gaggggcgga cgttgacact tgaggggccg     660

actcacccgg cgcggcgttg acagatgagg ggcaggctcg atttcggccg gcgacgtgga     720

gctggccagc ctcgcaaatc ggcgaaaacg cctgatttta cgcgagtttc ccacagatga     780

tgtggacaag cctggggata agtgccctgc ggtattgaca cttgaggggc gcgactactg     840

acagatgagg ggcgcgatcc ttgacacttg aggggcagag tgctgacaga tgaggggcgc     900

acctattgac atttgagggg ctgtccacag gcagaaaatc cagcatttgc aagggtttcc     960

gcccgttttt cggccaccgc taacctgtct tttaacctgc ttttaaacca atatttataa    1020

accttgtttt taaccagggc tgcgccctgt gcgcgtgacc gcgcacgccg aaggggggtg    1080

cccccccttc tcgaaccctc ccggcccgct aacgcgggcc tcccatcccc ccaggggctg    1140

cgcccctcgg ccgcgaacgg cctcacccca aaaatggcag cgctggccaa ttcgtgcgcg    1200

gaacccctat ttgtttattt ttctaaatac attcaaatat gtatccgctc atgagacaat    1260

aaccctgata aatgcttcaa taatattgaa aaaggaagag tatggctaaa atgagaatat    1320

caccggaatt gaaaaaactg atcgaaaaat accgctgcgt aaaagatacg gaaggaatgt 1380

ctcctgctaa ggtatataag ctggtgggag aaaatgaaaa cctatattta aaaatgacgg 1440

acagccggta taaagggacc acctatgatg tggaacggga aaaggacatg atgctatggc 1500

tggaaggaaa gctgcctgtt ccaaaggtcc tgcactttga acggcatgat ggctggagca 1560

atctgctcat gagtgaggcc gatggcgtcc tttgctcgga agagtatgaa gatgaacaaa 1620

gccctgaaaa gattatcgag ctgtatgcgg agtgcatcag gctctttcac tccatcgaca 1680

tatcggattg tccctatacg aatagcttag acagccgctt agccgaattg gattacttac 1740

tgaataacga tctggccgat gtggattgcg aaaactggga agaagacact ccatttaaag 1800

atccgcgcga gctgtatgat tttttaaaga cggaaaagcc cgaagaggaa cttgtctttt 1860

cccacggcga cctgggagac agcaacatct ttgtgaaaga tggcaaagta agtggcttta 1920

ttgatcttgg gagaagcggc agggcggaca agtggtatga cattgccttc tgcgtccggt 1980

cgatcaggga ggatatcggg gaagaacagt atgtcgagct attttttgac ttactgggga 2040

tcaagcctga ttgggagaaa ataaaatatt atattttact ggatgaattg ttttagctgt 2100

cagaccaagt ttactcatat atactttaga ttgatttaaa acttcatttt taatttaaaa 2160

ggatctaggt gaagatcctt tttgataatc tcatgaccaa aatcccttaa cgtgagtttt 2220

cgttccactg agcgtcagac cccgtagaaa agatcaaagg atcttcttga gatccttttt 2280

ttctgcgcgt aatctgctgc ttgcaaacaa aaaaaccacc gctaccagcg gtggtttgtt 2340

tgccggatca agagctacca actctttttc cgaaggtaac tggcttcagc agagcgcaga 2400

taccaaatac tgtccttcta gtgtagccgt agttaggcca ccacttcaag aactctgtag 2460

caccgcctac atacctcgct ctgctaatcc tgttaccagt ggctgctgcc agtggcgata 2520

agtcgtgtct taccgggttg gactcaagac gatagttacc ggataaggcg cagcggtcgg 2580

gctgaacggg gggttcgtgc acacagccca gcttggagcg aacgacctac accgaactga 2640

gatacctaca gcgtgagcta tgagaaagcg ccacgcttcc cgaagggaga aaggcggaca 2700

ggtatccggt aagcggcagg gtcggaacag gagagcgcac gagggagctt ccagggggaa 2760

acgcctggta tctttatagt cctgtcgggt ttcgccacct ctgacttgag cgtcgatttt 2820

tgtgatgctc gtcagggggg cggagcctat ggaaaaacgc cagcaacgcg gcctttttac 2880

ggttcctggc agatcctaga tgtggcgcaa cgatgccggc gacaagcagg agcgcaccga 2940

cttcttccgc atcaagtgtt ttggctctca ggccgaggcc cacggcaagt atttgggcaa 3000

ggggtcgctg gtattcgtgc agggcaagat tcggaatacc aagtacgaga aggacggcca 3060

gacggtctac gggaccgact tcattgccga taaggtggat tatctggaca ccaaggcacc 3120

aggcgggtca aatcaggaat aagggcacat tgccccggcg tgagtcgggg caatcccgca 3180

aggagggtga atgaatcgga cgtttgaccg gaaggcatac aggcaagaac tgatcgacgc 3240

ggggttttcc gccgaggatg ccgaaaccat cgcaagccgc accgtcatgc gtgcgccccg 3300
cgaaaccttc cagtccgtcg gctcgatggt ccagcaagct acggccaaga tcgagcgcga 3360
cagcgtgcaa ctggctcccc ctgccctgcc cgcgccatcg gccgccgtgg agcgttcgcg 3420
tcgtctcgaa caggaggcgg caggtttggc gaagtcgatg accatcgaca cgcgaggaac 3480
tatgacgacc aagaagcgaa aaaccgccgg cgaggacctg gcaaaacagg tcagcgaggc 3540
caagcaggcc gcgttgctga aacacacgaa gcagcagatc aaggaaatgc agctttcctt 3600
gttcgatatt gcgccgtggc cggacacgat gcgagcgatg ccaaacgaca cggcccgctc 3660
tgccctgttc accacgcgca acaagaaaat cccgcgcgag gcgctgcaaa acaaggtcat 3720
tttccacgtc aacaaggacg tgaagatcac ctacaccggc gtcgagctgc gggccgacga 3780
tgacgaactg gtgtggcagc aggtgttgga gtacgcgaag cgcaccccta tcggcgagcc 3840
gatcaccttc acgttctacg agctttgcca ggacctgggc tggtcgatca atggccggta 3900
ttacacgaag gccgaggaat gcctgtcgcg cctacaggcg acggcgatgg gcttcacgtc 3960
cgaccgcgtt gggcacctgg aatcggtgtc gctgctgcac cgcttccgcg tcctggaccg 4020
tggcaagaaa acgtcccgtt gccaggtcct gatcgacgag gaaatcgtcg tgctgtttgc 4080
tggcgaccac tacacgaaat tcatatggga gaagtaccgc aagctgtcgc cgacggcccg 4140
acggatgttc gactatttca gctcgcaccg ggagccgtac ccgctcaagc tggaaacctt 4200
ccgcctcatg tgcggatcgg attccacccg cgtgaagaag tggcgcgagc aggtcggcga 4260
agcctgcgaa gagttgcgag gcagcggcct ggtggaacac gcctgggtca atgatgacct 4320
ggtgcattgc aaacgctagg gccttgtggg gtcagttccg gctgggggtt cagcagccag 4380
cgcctgatct ggggaaccct gtggttggca catacaaatg gacgaacgga taaacctttt 4440
cacgcccttt taaatatccg attattctaa taaacgctct tttctcttag gtttacccgc 4500
caatatatcc tgtcaaacac tgatagttta aactgaaggc gggaaacgac aatctgatct 4560
aagctagctt ggaattggta ccacgcgttt cgacaaaatt tagaacgaac ttaattatga 4620
tctcaaatac attgatacat atctcatcta gatctaggtt atcattatgt aagaaagttt 4680
tgacgaatat ggcacgacaa aatggctaga ctcgatgtaa ttggtatctc aactcaacat 4740
tatacttata ccaaacatta gttagacaaa atttaaacaa ctatttttta tgtatgcaag 4800
agtcagcata tgtataattg attcagaatc gttttgacga gttcggatgt agtagtagcc 4860
attatttaat gtacatacta atcgtgaata gtgaatatga tgaaacattg tatcttattg 4920
tataaatatc cataaacaca tcatgaaaga cactttcttt cacggtctga attaattatg 4980
atacaattct aatagaaaac gaattaaatt acgttgaatt gtatgaaatc taattgaaca 5040
agccaaccac gacgacgact aacgttgcct ggattgactc ggtttaagtt aaccactaaa 5100
aaaacggagc tgtcatgtaa cacgcggatc gagcaggtca cagtcatgaa gccatcaaag 5160

caaaagaact aatccaaggg ctgagatgat taattagttt aaaaattagt taacacgagg 5220

gaaaaggctg tctgacagcc aggtcacgtt atctttacct gtggtcgaaa tgattcgtgt 5280

ctgtcgattt taattatttt tttgaaaggc cgaaaataaa gttgtaagag ataaacccgc 5340

ctatataaat tcatatattt tcctctccgc tttgaagttt tagttttatt gcaacaacaa 5400

caacaaatta caataacaac aaacaaaata caaacaacaa caacatggca caatttcaac 5460

aaacaattga catgcaaact ctccaagccg ctgcgggacg caacagcttg gtgaatgatt 5520

tggcatctcg tcgcgtttac gataatgcag tcgaggagct gaatgctcgt tccagacgtc 5580

ccaaggtaaa acaacatttc attcacatat atgaatactt ttgtcattga gtacgaagaa 5640

gacacttact acttgttgat gaaagtttcc gcctttatac ttatctatat cattttcatc 5700

atttcaaact agtatgaaat taggtgatgt ttatatgata tcatggaaca ttaatctata 5760

gggaaactgt tttgagttag ttttgtataa tatttttccc tgtttgatgt taggttcatt 5820

tctccaaggc agtgtctacg gaacagacac tgattgcaac aaacgcatat ccggagttcg 5880

agatttcctt tactcatacg caatccgctg tgcactcctt ggccggaggc cttcggtcac 5940

ttgagttgga gtatctcatg atgcaagttc cgttcggctc tctgacctac gacatcggcg 6000

gaaacttctc cgcgcacctc ttcaaaggta attttctttc tctactcaat tttctccaag 6060

atccaatatt tgaagactga tctatagtta aaattaatct ctactccatt cttgttacct 6120

caggtcgcga ttacgttcac tgctgcatgc ctaatctgga tgtacgtgac attgctcgcc 6180

atgaaggaca caaggaagct atttacagtt atgtgaatcg tttgaaaagg cagcagcgtc 6240

ctgtgcctga ataccagagg gcagctttca acaactacgc tgagaacccg cacttcgtcc 6300

attgcgacaa acctttccaa cagtgtgaat tgacgacagc gtatggcact gacacctacg 6360

ctgtagctct ccatagcatt tatgatatcc ctgttgagga gttcggttct gcgctactca 6420

ggaagaatgt gaaaacttgt ttcgcggcct ttcatttcca tgagaatatg cttctagatt 6480

gtgatacagt cacactcgat gagattggag ctacgttcca gaaatcaggt aacattcctt 6540

agttaccttt cttttctttt tccatcataa gtttatagat tgtacatgct ttgagatttt 6600

tctttgcaaa caatctcagg tgataacctg agcttcttct tccataatga gagcactctc 6660

aattacaccc acagcttcag caacatcatc aagtacgtgt gcaagacgtt cttccctgct 6720

agtcaacgct tcgtgtacca caaggagttc ctggtcacta gagtcaacac ttggtactgc 6780

aagttcacga gagtggatac gttcactctg ttccgtggtg tgtaccacaa caatgtggat 6840

tgcgaagagt tttacaaggc tatggacgat gcgtggcact acaaaaagac gttagcaatg 6900

cttaatgccg agaggaccat cttcaaggat aacgctgcgt taaacttctg gttcccgaag 6960

gtgctcttga aattggaagt cttcttttgt tgtctaaacc tatcaatttc tttgcggaaa 7020

tttatttgaa gctgtagagt taaaattgag tcttttaaac ttttgtaggt gagagacatg 7080

gttatcgtcc ctctctttga cgcttctatc acaactggta ggatgtctag gagagaggtt 7140
atggtgaaca aggacttcgt ctacacggtc ctaaatcaca tcaagaccta tcaagctaag 7200
gcactgacgt acgcaaacgt gctgagcttc gtggagtcta ttaggtctag agtgataatt 7260
aacggtgtca ctgccaggta agttgttact tatgattgtt ttcctctctg ctacatgtat 7320
tttgttgttc atttctgtaa gatataagaa ttgagttttc ctctgatgat attattaggt 7380
ctgaatggga cacagacaag gcaattctag gtccattagc aatgacattc ttcctgatca 7440
cgaagctggg tcatgtgcaa gatgaaataa tcctgaaaaa gttccagaag ttcgacagaa 7500
ccaccaatga gctgatttgg acaagtctct gcgatgccct gatgggggtt attccctcgg 7560
tcaaggagac gcttgtgcgc ggtggttttg tgaaagtagc agaacaagcc ttagagatca 7620
aggttagtat catatgaaga aatacctagt ttcagttgat gaatgctatt ttctgacctc 7680
agttgttctc ttttgagaat tatttctttt ctaatttgcc tgatttttct attaattcat 7740
taggttcccg agctatactg taccttcgcc gaccgattgg tactacagta caagaaggcg 7800
gaggagttcc aatcgtgtga tctttccaaa cctctagaag agtcagagaa gtactacaac 7860
gcattatccg agctatcagt gcttgagaat ctcgactctt ttgacttaga ggcgtttaag 7920
actttatgtc agcagaagaa tgtggacccg gatatggcag caaaggtaaa tcctggtcca 7980
cacttttacg ataaaaacac aagattttaa actatgaact gatcaataat cattcctaaa 8040
agaccacact tttgttttgt ttctaaagta atttttactg ttataacagg tggtcgtagc 8100
aatcatgaag tcagaattga cgttgccttt caagaaacct acagaagagg aaatctcgga 8160
gtcgctaaaa ccaggagagg ggtcgtgtgc agagcataag gaagtgttga gcttacaaaa 8220
tgatgctccg ttcccgtgtg tgaaaaatct agttgaaggt tccgtgccgg cgtatggaat 8280
gtgtcctaag ggtggtggtt tcgacaaatt ggatgtggac attgctgatt tccatctcaa 8340
gagtgtagat gcagttaaaa agggaactat gatgtctgcg gtgtacacag ggtctatcaa 8400
agttcaacaa atgaagaact acatagatta cttaagtgcg tcgctggcag ctacagtctc 8460
aaacctctgc aaggtaagag gtcaaaaggt ttccgcaatg atccctcttt ttttgtttct 8520
ctagtttcaa gaatttgggt atatgactaa cttctgagtg ttccttgatg catatttgtg 8580
atgagacaaa tgtttgttct atgttttagg tgcttagaga tgttcacggc gttgacccag 8640
agtcacagga gaaatctgga gtgtgggatg ttaggagagg acgttggtta cttaaaccta 8700
atgcgaaaag tcacgcgtgg ggtgtggcag aagacgccaa ccacaagttg gttattgtgt 8760
tactcaactg ggatgacgga aagccggttt gtgatgagac atggttcagg gtggcggtgt 8820
caagcgattc cttgatatat tcggatatgg gaaaacttaa gacgctcacg tcttgcagtc 8880
caaatggtga gccaccggag cctaacgcca aagtaatttt ggtcgatggt gttcccggtt 8940
gtggaaaaac gaaggagatt atcgaaaagg taagttctgc atttggttat gctccttgca 9000

ttttaggtgt tcgtcgctct tccatttcca tgaatagcta agattttttt tctctgcatt 9060
cattcttctt gcctcagttc taactgtttg tggtattttt gttttaatta ttgctacagg 9120
taaacttctc tgaagacttg attttagtcc ctgggaagga agcttctaag atgatcatcc 9180
ggagggccaa ccaagctggt gtgataagag cggataagga caatgttaga acggtggatt 9240
ccttcttgat gcatccttct agaagggtgt ttaagaggtt gtttatcgat gaaggactaa 9300
tgctgcatac aggttgtgta aatttcctac tgctgctatc tcaatgtgac gtcgcatatg 9360
tgtatgggga cacaaagcaa attccgttca tttgcagagt cgcgaacttt ccgtatccag 9420
cgcattttgc aaaactcgtc gctgatgaga aggaagtcag aagagttacg ctcaggtaaa 9480
gcaactgtgt tttaatcaat ttcttgtcag gatatatgga ttataactta atttttgaga 9540
aatctgtagt atttggcgtg aaatgagttt gctttttggt ttctcccgtg ttataggtgc 9600
ccggctgatg ttacgtattt ccttaacaag aagtatgacg gggcggtgat gtgtaccagc 9660
gcggtagaga gatccgtgaa ggcagaagtg gtgagaggaa agggtgcatt gaacccaata 9720
accttaccgt tggagggtaa aattttgacc ttcacacaag ctgacaagtt cgagttactg 9780
gagaagggtt acaaggtaaa gtttccaact ttcctttacc atatcaaact aaagttcgaa 9840
actttttatt tgatcaactt caaggccacc cgatctttct attcctgatt aatttgtgat 9900
gaatccatat tgacttttga tggttacgca ggatgtgaac actgtgcacg aggtgcaagg 9960
ggagacgtac gagaagactg ctattgtgcg cttgacatca actccgttag agatcatatc 10020
gagtgcgtca cctcatgttt tggtggcgct gacaagacac acaacgtgtt gtaaatatta 10080
caccgttgtg ttggacccga tggtgaatgt gatttcagaa atggagaagt tgtccaattt 10140
ccttcttgac atgtatagag ttgaagcagg tctgtctttc ctatttcata tgtttaatcc 10200
taggaatttg atcaattgat tgtatgtatg tcgatcccaa gactttcttg ttcacttata 10260
tcttaactct ctctttgctg tttcttgcag gtgtccaata gcaattacaa atcgatgcag 10320
tattcagggg acagaacttg tttgttcaga cgcccaagtc aggagattgg cgagatatgc 10380
aattttacta tgacgctctt cttcccggaa acagtactat tctcaatgaa tttgatgctg 10440
ttacgatgaa tttgagggat atttccttaa acgtcaaaga ttgcagaatc gacttctcca 10500
aatccgtgca acttcctaaa gaacaaccta ttttcctcaa gcctaaaata agaactgcgg 10560
cagaaatgcc gagaactgca ggtaaaatat tggatgccag acgatattct ttcttttgat 10620
ttgtaacttt ttcctgtcaa ggtcgataaa ttttattttt tttggtaaaa ggtcgataat 10680
ttttttttgg agccattatg taattttcct aattaactga accaaaatta tacaaaccag 10740
gtttgctgga aaatttggtt gcaatgatca aaagaaacat gaatgcgccg gatttgacag 10800
ggacaattga cattgaggat actgcatctc tggtggttga aaagttttgg gattcgtatg 10860
ttgacaagga atttagtgga acgaacgaaa tgaccatgac aagggagagc ttctccaggt 10920

aaggacttct catgaatatt agtggcagat tagtgttgtt aaagtctttg gttagataat 10980
cgatgcctcc taattgtcca tgttttactg gttttctaca attaaaggtg gctttcgaaa 11040
caagagtcat ctacagttgg tcagttagcg gactttaact ttgtggattt gccggcagta 11100
gatgagtaca agcatatgat caagagtcaa ccaaagcaaa agttagactt gagtattcaa 11160
gacgaatatc ctgcattgca gacgatagtc taccattcga aaaagatcaa tgcgattttc 11220
ggtccaatgt tttcagaact tacgaggatg ttactcgaaa ggattgactc ttcgaagttt 11280
ctgttctaca ccagaaagac acctgcacaa atagaggact tcttttctga cctagactca 11340
acccaggcga tggaaattct ggaactcgac atttcgaagt acgataagtc acaaaacgag 11400
ttccattgtg ctgtagagta caagatctgg gaaaagttag gaattgatga gtggctagct 11460
gaggtctgga aacaaggtga gttcctaagt tccatttttt tgtaatcctt caatgttatt 11520
ttaacttttc agatcaacat caaaattagg ttcaattttc atcaaccaaa taatattttt 11580
catgtatata taggtcacag aaaaacgacc ttgaaagatt atacggccgg aatcaaaaca 11640
tgtctttggt atcaaaggaa aagtggtgat gtgacaacct ttattggtaa taccatcatc 11700
attgccgcat gtttgagctc aatgatcccc atggacaaag tgataaaggc agctttttgt 11760
ggagacgata gcctgattta cattcctaaa ggtttagact tgcctgatat tcaggcgggc 11820
gcgaacctca tgtggaactt cgaggccaaa ctcttcagga agaagtatgg ttacttctgt 11880
ggtcgttatg ttattcacca tgatagagga gccattgtgt attacgatcc gcttaaacta 11940
atatctaagt taggttgtaa acatattaga gatgttgttc acttagaaga gttacgcgag 12000
tctttgtgtg atgtagctag taacttaaat aattgtgcgt atttttcaca gttagatgag 12060
gccgttgccg aggttcataa gaccgcggta ggcggttcgt ttgctttttg tagtataatt 12120
aagtatttgt cagataagag attgtttaga gatttgttct ttgtttgata atgtcgatag 12180
tctcgtacga acctaaggtg agtgatttcc tcaatctttc gaagaaggaa gagatcttgc 12240
cgaaggctct aacgaggtta aaaaccgtgt ctattagtac taaagatatt atatctgtca 12300
aggagtcgga gactttgtgt gatatagatt tgttaatcaa tgtgccatta gataagtata 12360
gatatgtggg tatcctagga gccgttttta ccggagagtg gctagtgcca gacttcgtta 12420
aaggtggagt gacgataagt gtgatagata agcgtctggt gaactcaaag gagtgcgtga 12480
ttggtacgta cagagccgca gccaagagta agaggttcca gttcaaattg gttccaaatt 12540
actttgtgtc caccgtggac gcaaagagga agccgtggca ggtaaggatt tttatgatat 12600
agtatgctta tgtattttgt actgaaagca tatcctgctt cattgggata ttactgaaag 12660
catttaacta catgtaaact cacttgatga tcaataaact tgattttgca ggttcatgtt 12720
cgtatacaag acttgaagat tgaggcgggt tggcagccgt tagctctgga agtagtttca 12780
gttgctatgg tcaccaataa cgttgtcatg aagggtttga gggaaaaggt cgtcgcaata 12840

aatgatccgg acgtcgaagg tttcgaaggt aagccatctt cctgcttatt tttataatga 12900

acatagaaat aggaagttgt gcagagaaac taattaacct gactcaaaat ctaccctcat 12960

aattgttgtt tgatattggt cttgtatttt gcaggtgtgg ttgacgaatt cgtcgattcg 13020

gttgcagcat ttaaagcggt tgacaacttt aaaagaagga aaaagaaggt tgaagaaaag 13080

ggtgtagtaa gtaagtataa gtacagaccg gagaagtacg ccggtcctga ttcgtttaat 13140

ttgaaagaag aaaacgtctt acaacattac aaacccgaat cagtaccagt atttcgataa 13200

gaaacaagaa atggggaagc aaatggccgc cctgtgtggc tttctcctcg tggcgttgct 13260

ctggctcacg cccgacgtcg cgcatggtat gtctgcaaac caggaggaag acaagaagcc 13320

aggagacgga ggagctcaca tcaatctcaa agtcaaggga caggtatctc tctttctcct 13380

tctcatcctt gtgtgttctt gtgaatgttt gggtttctga tttcgtgtag ctgcgattag 13440

ggtttttcat tctccaattt ggtttaattt tagggtttcg tactactagc tcagtcttaa 13500

tggtcttctt cctcttttgt tttacaggtt taaagtatcc tgctttatgt ttacacgttt 13560

gcttgttttt gctaattgtt tatcaaattt ctgaaattat ataagtcttg tttaggtgta 13620

agttgttatt gagcttttgg ttcctgttgt tgtttgaggg ttttatagtt ttggaaggga 13680

taagtttact tagttgttga atgttctaaa gactgtgaac gatgctgtct ctgtactaag 13740

ttgttatata tcttctgatg aagttgtagt ctctgtacta agttttcaat tagtttggct 13800

gatgtttgtg cttcaaattc tcatagacga agccttaaac tgaatgtttg ttgatatgtg 13860

tttaattttt cgactctttc aggttactac caaaagagag ccatgtgatt tagttattgt 13920

ttcatatgcg gtctctaacg tttattgttc cttctatatg tttgttctat aggatggaaa 13980

cgaggttttc tttaggatca agagaagcac tcagctcaag aagctgatga atgcttactg 14040

tgaccggcaa tctgtggaca tgaactccat tgctttcttg tttgatgggc gtcgtcttcg 14100

tgctgagcaa actcccgatg aggtataaca ttcattctac atgctttatt tcttaccttt 14160

tgaagtttat agtttctgat actaataatt cttgacacta cagcttgaca tggaggatgg 14220

tgatgagatc gatgcgatgc ttcatcagac tggaggtttc cccgtggacg atgatgacaa 14280

gatcgtgggc ggctacacct gtggggcaaa tactgtcccc taccaagtgt ccctgaactc 14340

tggctaccac ttctgcgggg gctccctcat caacagccag tgggtggtgt ctgcggctca 14400

ctgctacaag tccggaatcc aagtgcgtct gggagaagac aacattaatg tcgttgaggg 14460

caatgagcaa ttcatcagcg catccaagag catcgtccat cccagctaca actcaaacac 14520

cttaaacaac gacatcatgc tgattaaact gaaatcagct gccagtctca acagccgagt 14580

agcctctatc tctctgccaa catcctgtgc ctctgctggc acccagtgtc tcatctctgg 14640

ctggggcaac accaaaagca gtggcaccag ctaccctgat gtcctgaagt gtctgaaggc 14700

tcccatccta tcagacagct cttgcaaaag tgcctaccca ggccagatca ccagcaacat 14760

gttctgtgcg ggctacctgg agggcggaaa ggactcctgc cagggtgact ccggtggccc 14820

tgtggtctgc agtggaaagc tccagggcat tgtctcctgg ggctctggct gcgctcagaa 14880

aaacaagcct ggtgtctaca ccaaggtctg caactacgtg agctggatta agcagaccat 14940

cgcctccaac taaagcttac tagagcgtgg tgcgcacgat agcgcatagt gtttttctct 15000

ccacttgaat cgaagagata gacttacggt gtaaatccgt aggggtggcg taaaccaaat 15060

tacgcaatgt tttgggttcc atttaaatcg aaacccctta tttcctggat cacctgttaa 15120

cgcacgtttg acgtgtatta cagtgggaat aagtaaaagt gagaggttcg aatcctccct 15180

aacc 15184

<210> 2
<211> 78
<212> DNA
<213> Artificial Sequence

<220>
<223> sequence coding for apoplast signal peptide

<400> 2
atggggaagc aaatggccgc cctgtgtggc tttctcctcg tggcgttgct ctggctcacg 60

cccgacgtcg cgcatggt 78

<210> 3
<211> 180
<212> DNA
<213> Artificial Sequence

<220>
<223> sequence coding for plastid signal peptide

<400> 3
atggcttctt ctatgctttc ttctgctgct gttgttgcta ctcgtgctag tgctgctcaa 60

gctagtatgg ttgctccttt tactggactt aagtctgctg cttcttttcc tgttactaga 120

aagcaaaaca accttgatat tacttctatt gctagtaacg gaggaagagt ccaatgcgca 180

<210> 4
<211> 243
<212> PRT
<213> bos taurus

<400> 4

Phe Ile Phe Leu Ala Leu Leu Gly Ala Ala Val Ala Phe Pro Val Asp
1               5                   10                  15

Asp Asp Asp Lys Ile Val Gly Gly Tyr Thr Cys Gly Ala Asn Thr Val
            20                  25                  30

Pro Tyr Gln Val Ser Leu Asn Ser Gly Tyr His Phe Cys Gly Gly Ser

        35                  40                  45

Leu Ile Asn Ser Gln Trp Val Val Ser Ala Ala His Cys Tyr Lys Ser
    50                  55                  60

Gly Ile Gln Val Arg Leu Gly Glu Asp Asn Ile Asn Val Val Glu Gly
65                  70                  75                  80

Asn Glu Gln Phe Ile Ser Ala Ser Lys Ser Ile Val His Pro Ser Tyr
                85                  90                  95

Asn Ser Asn Thr Leu Asn Asn Asp Ile Met Leu Ile Lys Leu Lys Ser
            100                 105                 110

Ala Ala Ser Leu Asn Ser Arg Val Ala Ser Ile Ser Leu Pro Thr Ser
        115                 120                 125

Cys Ala Ser Ala Gly Thr Gln Cys Leu Ile Ser Gly Trp Gly Asn Thr
    130                 135                 140

Lys Ser Ser Gly Thr Ser Tyr Pro Asp Val Leu Lys Cys Leu Lys Ala
145                 150                 155                 160

Pro Ile Leu Ser Asp Ser Ser Cys Lys Ser Ala Tyr Pro Gly Gln Ile
                165                 170                 175

Thr Ser Asn Met Phe Cys Ala Gly Tyr Leu Glu Gly Gly Lys Asp Ser
            180                 185                 190

Cys Gln Gly Asp Ser Gly Gly Pro Val Val Cys Ser Gly Lys Leu Gln
        195                 200                 205

Gly Ile Val Ser Trp Gly Ser Gly Cys Ala Gln Lys Asn Lys Pro Gly
    210                 215                 220

Val Tyr Thr Lys Val Cys Asn Tyr Val Ser Trp Ile Lys Gln Thr Ile
225                 230                 235                 240

Ala Ser Asn

<210> 5
<211> 825
<212> DNA
<213> Bos taurus

<400> 5
cttcatcttt ctggctctct tgggagccgc tgttgctttc cccgtggacg atgatgacaa 60

gatcgtgggc ggctacacct gtggggcaaa tactgtcccc taccaagtgt ccctgaactc 120

tggctaccac ttctgcgggg gctccctcat caacagccag tgggtggtgt ctgcggctca 180

ctgctacaag tccggaatcc aagtgcgtct gggagaagac aacattaatg tcgttgaggg 240

caatgagcaa ttcatcagcg catccaagag tatcgtccat cccagctaca actcaaacac 300

cttaaacaac gacatcatgc tgattaaact gaaatcagct gccagtctca acagccgagt 360

agcctctatc tctctgccaa catcctgtgc ctctgctggc acccagtgtc tcatctctgg 420

ctggggcaac accaaaagca gtggcaccag ctaccctgat gtcctgaagt gtctgaaggc 480

tcccatccta tcagacagct cttgcaaaag tgcctaccca ggccagatca ccagcaacat 540

gttctgtgcg ggctacctgg agggcggaaa ggactcctgc cagggtgact ccggtggccc 600

tgtggtctgc agtggaaagc tccagggcat tgtctcctgg ggctctggct gcgctcagaa 660

aaacaagcct ggtgtctaca ccaaggtctg caactacgtg agctggatta agcagaccat 720

cgcctccaac taaatagctt catctcttca tgaccctctc tgctagccag cttcaccttc 780

ctcccatcct gaacgcacta cttaaataaa atcatttata aaacc 825

<210> 6
<211> 1060
<212> DNA
<213> Artificial Sequence

<220>
<223> synthetic EPB-2 gene

<400> 6
ggtctcaagg tattcctatg gaagataagg atcttgagtc tgaagaggct ctttgggatc 60

tttatgagag gtggcagtct gctcatagag tgagaaggca tcatgctgag aaacatagaa 120

gattcggtac tttcaagtct aatgctcatt tcattcattc tcataataag aggggtgatc 180

atccttacag gcttcatctt aatagattcg gtgatatgga tcaggctgag ttcagggcta 240

ctttcgttgg tgatcttaga agggatactc cttctaagcc tccttctgtg cctggtttta 300

tgtacgctgc tcttaatgtg tctgatcttc ctccatctgt tgattggagg cagaagggtg 360

ctgttactgg agttaaggat cagggaaagt gtggttcttg ctgggctttc tctactgttg 420

tttctgtgga gggtatcaat gctattagga ctggttctct tgtgtctctt tctgagcaag 480

agcttattga ttgcgatact gctgataatg atggttgcca gggtggtctt atggataatg 540

ctttcgagta cattaagaac aatggtggtc ttattactga ggctgcttac ccttatagag 600

ctgctagggg aacttgtaac gttgctaggg ctgctcagaa ttctcctgtg gtggtgcata 660

ttgatggtca tcaggatgtg cctgctaatt ctgaagagga tttggctagg gctgttgcta 720

atcagcctgt ttctgttgct gttgaggctt ctggaaaggc tttcatgttc tactctgagg 780

gagtttttac tggtgagtgc ggtactgaac ttgatcatgg tgttgctgtt gttggttacg 840

gtgttgctga agatggaaag gcttactgga ctgtgaagaa ttcttgggga ccttcttggg 900

gtgaacaggg ttacattagg gtggagaagg attctggtgc ttctggtggt ctttgcggta     960

ttgcaatgga agctagttac cctgttaaga cttactctaa gcctaagcct actcctagaa    1020

gagctttggg tgctagagag tctctttaag ctttgagacc                          1060

<210>  7
<211>  345
<212>  PRT
<213>  Artificial Sequence

<220>
<223>  expression porduct of synthetic EPB-2 gene

<400>  7

Ile Pro Met Glu Asp Lys Asp Leu Glu Ser Glu Glu Ala Leu Trp Asp
1               5                   10                  15

Leu Tyr Glu Arg Trp Gln Ser Ala His Arg Val Arg Arg His His Ala
            20                  25                  30

Glu Lys His Arg Arg Phe Gly Thr Phe Lys Ser Asn Ala His Phe Ile
        35                  40                  45

His Ser His Asn Lys Arg Gly Asp His Pro Tyr Arg Leu His Leu Asn
    50                  55                  60

Arg Phe Gly Asp Met Asp Gln Ala Glu Phe Arg Ala Thr Phe Val Gly
65                  70                  75                  80

Asp Leu Arg Arg Asp Thr Pro Ser Lys Pro Pro Ser Val Pro Gly Phe
                85                  90                  95

Met Tyr Ala Ala Leu Asn Val Ser Asp Leu Pro Pro Ser Val Asp Trp
            100                 105                 110

Arg Gln Lys Gly Ala Val Thr Gly Val Lys Asp Gln Gly Lys Cys Gly
        115                 120                 125

Ser Cys Trp Ala Phe Ser Thr Val Val Ser Val Glu Gly Ile Asn Ala
    130                 135                 140

Ile Arg Thr Gly Ser Leu Val Ser Leu Ser Glu Gln Glu Leu Ile Asp
145                 150                 155                 160

Cys Asp Thr Ala Asp Asn Asp Gly Cys Gln Gly Gly Leu Met Asp Asn
                165                 170                 175

Ala Phe Glu Tyr Ile Lys Asn Asn Gly Gly Leu Ile Thr Glu Ala Ala
            180                 185                 190

Tyr Pro Tyr Arg Ala Ala Arg Gly Thr Cys Asn Val Ala Arg Ala Ala
        195                 200                 205

Gln Asn Ser Pro Val Val Val His Ile Asp Gly His Gln Asp Val Pro
    210                 215                 220

Ala Asn Ser Glu Glu Asp Leu Ala Arg Ala Val Ala Asn Gln Pro Val
225                 230                 235                 240

Ser Val Ala Val Glu Ala Ser Gly Lys Ala Phe Met Phe Tyr Ser Glu
                245                 250                 255

Gly Val Phe Thr Gly Glu Cys Gly Thr Glu Leu Asp His Gly Val Ala
            260                 265                 270

Val Val Gly Tyr Gly Val Ala Glu Asp Gly Lys Ala Tyr Trp Thr Val
        275                 280                 285

Lys Asn Ser Trp Gly Pro Ser Trp Gly Glu Gln Gly Tyr Ile Arg Val
    290                 295                 300

Glu Lys Asp Ser Gly Ala Ser Gly Gly Leu Cys Gly Ile Ala Met Glu
305                 310                 315                 320

Ala Ser Tyr Pro Val Lys Thr Tyr Ser Lys Pro Lys Pro Thr Pro Arg
                325                 330                 335

Arg Ala Leu Gly Ala Arg Glu Ser Leu
            340                 345

<210>  8
<211>  30
<212>  DNA
<213>  Artificial Sequence

<220>
<223>  PCR primer

<400>  8
ggtctcaagg tttccccgtg gacgatgatg                                        30

<210>  9
<211>  30
<212>  DNA
<213>  Artificial Sequence

<220>
<223>  PCR primer

<400>  9
ggtctcaagg tatcgtgggc ggctacacct                                        30

<210>  10
<211>  30
<212>  DNA
<213>  Artificial Sequence

<220>
<223>  PCR primer

<400>  10
ggtctcatcc ggacttgtag cagtgagccg                                        30


<210>  11
<211>  27
<212>  DNA
<213>  Artificial Sequence

<220>
<223>  PCR primer

<400>  11
ggtctcacgg aatccaagtg cgtctgg                                           27


<210>  12
<211>  29
<212>  DNA
<213>  Artificial Sequence

<220>
<223>  PCR primer

<400>  12
ggtctcatgc cactgctttt ggtgttgcc                                         29


<210>  13
<211>  29
<212>  DNA
<213>  Artificial Sequence

<220>
<223>  PCR primer

<400>  13
ggtctcaggc accagctacc ctgatgtcc                                         29


<210>  14
<211>  26
<212>  DNA
<213>  Artificial Sequence

<220>
<223>  PCR primer

<400>  14
ggtctcactg gcaggagtcc tttccg                                            26


<210>  15
<211>  30

<212>  DNA
<213>  Artificial Sequence

<220>
<223>  PCR primer

<400>  15
ggtctcacca gggtgactcc ggtggccctg                                        30


<210>  16
<211>  33
<212>  DNA
<213>  Artificial Sequence

<220>
<223>  PCR primer

<400>  16
ggtctcaagc tttagttgga ggcgatggtc tgc                                    33


<210>  17
<211>  95
<212>  PRT
<213>  Mus musculus

<400>  17

Met Ala Asp Glu Lys Pro Lys Glu Gly Val Lys Thr Glu Asn Asn Asp
1               5                   10                  15

His Ile Asn Leu Lys Val Ala Gly Gln Asp Gly Ser Val Val Gln Phe
            20                  25                  30

Lys Ile Lys Arg His Thr Pro Leu Ser Lys Leu Met Lys Ala Tyr Cys
        35                  40                  45

Glu Arg Gln Gly Leu Ser Met Arg Gln Ile Arg Phe Arg Phe Asp Gly
    50                  55                  60

Gln Pro Ile Asn Glu Thr Asp Thr Pro Ala Gln Leu Glu Met Glu Asp
65                  70                  75                  80

Glu Asp Thr Ile Asp Val Phe Gln Gln Gln Thr Gly Gly Val Tyr
                85                  90                  95


<210>  18
<211>  82
<212>  PRT
<213>  Mus musculus

<400>  18

Met Ser Glu Glu Lys Pro Lys Glu Gly Val Lys Thr Glu Asn Asp His
1               5                   10                  15

Ile Asn Leu Lys Val Ala Gly Gln Asp Gly Ser Val Val Gln Phe Lys
            20                  25                  30

Ile Lys Arg His Thr Pro Leu Ser Lys Leu Met Lys Ala Tyr Cys Glu
        35                  40                  45

Arg Gln Gly Leu Ser Met Arg Gln Ile Arg Phe Arg Phe Asp Gly Gln
    50                  55                  60

Pro Ile Asn Glu Thr Asp Thr Pro Ala Gln Phe Leu Ala Leu Thr Ile
65                  70                  75                  80

Leu Leu

<210>  19
<211>  101
<212>  PRT
<213>  Mus musculus

<400>  19

<210>  20
<211>  100
<212>  PRT
<213>  Oryza sativa

<400>  20

Met Ser Ala Ala Gly Glu Glu Asp Lys Lys Pro Ala Gly Gly Glu Gly
1               5                   10                  15

Gly Gly Ala His Ile Asn Leu Lys Val Lys Gly Gln Asp Gly Asn Glu
            20                  25                  30

Val Phe Phe Arg Ile Lys Arg Ser Thr Gln Leu Lys Lys Leu Met Asn
        35                  40                  45

Ala Tyr Cys Asp Arg Gln Ser Val Asp Met Asn Ala Ile Ala Phe Leu
    50                  55                  60

Phe Asp Gly Arg Arg Leu Arg Gly Glu Gln Thr Pro Asp Glu Leu Glu
65                  70                  75                  80

Met Glu Asp Gly Asp Glu Ile Asp Ala Met Leu His Gln Thr Gly Gly
                85                  90                  95

Cys Leu Pro Ala
            100

<210>  21
<211>  101
<212>  PRT
<213>  Saccharomyces cerevisiae

<400>  21

Met Ser Asp Ser Glu Val Asn Gln Glu Ala Lys Pro Glu Val Lys Pro
1               5                   10                  15

Glu Val Lys Pro Glu Thr His Ile Asn Leu Lys Val Ser Asp Gly Ser
            20                  25                  30

Ser Glu Ile Phe Phe Lys Ile Lys Lys Thr Thr Pro Leu Arg Arg Leu
        35                  40                  45

Met Glu Ala Phe Ala Lys Arg Gln Gly Lys Glu Met Asp Ser Leu Arg
    50                  55                  60

Phe Leu Tyr Asp Gly Ile Arg Ile Gln Ala Asp Gln Thr Pro Glu Asp
65                  70                  75                  80

Leu Asp Met Glu Asp Asn Asp Ile Ile Glu Ala His Arg Glu Gln Ile
                85                  90                  95

Gly Gly Ala Thr Tyr
            100

<210>  22
<211>  100
<212>  PRT

<213>  Arabidopsis thaliana

<400>  22

Met Ser Ala Asn Gln Glu Glu Asp Lys Lys Pro Gly Asp Gly Gly Ala
1               5                   10                  15

His Ile Asn Leu Lys Val Lys Gly Gln Asp Gly Asn Glu Val Phe Phe
            20                  25                  30

Arg Ile Lys Arg Ser Thr Gln Leu Lys Lys Leu Met Asn Ala Tyr Cys
        35                  40                  45

Asp Arg Gln Ser Val Asp Met Asn Ser Ile Ala Phe Leu Phe Asp Gly
    50                  55                  60

Arg Arg Leu Arg Ala Glu Gln Thr Pro Asp Glu Leu Asp Met Glu Asp
65                  70                  75                  80

Gly Asp Glu Ile Asp Ala Met Leu His Gln Thr Gly Gly Ser Gly Gly
                85                  90                  95

Gly Ala Thr Ala
            100

<210>  23
<211>  103
<212>  PRT
<213>  Arabidopsis thaliana

<400>  23

Met Ser Ala Thr Pro Glu Glu Asp Lys Lys Pro Asp Gln Gly Ala His
1               5                   10                  15

Ile Asn Leu Lys Val Lys Gly Gln Asp Gly Asn Glu Val Phe Phe Arg
            20                  25                  30

Ile Lys Arg Ser Thr Gln Leu Lys Lys Leu Met Asn Ala Tyr Cys Asp
        35                  40                  45

Arg Gln Ser Val Asp Phe Asn Ser Ile Ala Phe Leu Phe Asp Gly Arg
    50                  55                  60

Arg Leu Arg Ala Glu Gln Thr Pro Asp Glu Leu Glu Met Glu Asp Gly
65                  70                  75                  80

Asp Glu Ile Asp Ala Met Leu His Gln Thr Gly Gly Gly Ala Lys Asn
                85                  90                  95

Gly Leu Lys Leu Phe Cys Phe

            100

<210>  24
<211>  111
<212>  PRT
<213>  Arabidopsis thaliana

<400>  24

Met Ser Asn Pro Gln Asp Asp Lys Pro Ile Asp Gln Glu Gln Glu Ala
1               5                   10                  15

His Val Ile Leu Lys Val Lys Ser Gln Asp Gly Asp Glu Val Leu Phe
            20                  25                  30

Lys Asn Lys Lys Ser Ala Pro Leu Lys Lys Leu Met Tyr Val Tyr Cys
        35                  40                  45

Asp Arg Arg Gly Leu Lys Leu Asp Ala Phe Ala Phe Ile Phe Asn Gly
    50                  55                  60

Ala Arg Ile Gly Gly Leu Glu Thr Pro Asp Glu Leu Asp Met Glu Asp
65                  70                  75                  80

Gly Asp Val Ile Asp Ala Cys Arg Ala Met Ser Gly Gly Leu Arg Ala
                85                  90                  95

Asn Gln Arg Gln Trp Ser Tyr Met Leu Phe Asp His Asn Gly Leu
            100                 105                 110

<210>  25
<211>  114
<212>  PRT
<213>  Arabidopsis thaliana

<400>  25

Met Ser Thr Thr Ser Arg Val Gly Ser Asn Glu Val Lys Met Glu Gly
1               5                   10                  15

Gln Lys Arg Lys Val Val Ser Asp Pro Thr His Val Thr Leu Lys Val
            20                  25                  30

Lys Gly Gln Asp Glu Glu Asp Phe Arg Val Phe Trp Val Arg Arg Asn
        35                  40                  45

Ala Lys Leu Leu Lys Met Met Glu Leu Tyr Thr Lys Met Arg Gly Ile
    50                  55                  60

Glu Trp Asn Thr Phe Arg Phe Leu Phe Asp Gly Ser Arg Ile Arg Glu
65                  70                  75                  80

Tyr His Thr Pro Asp Glu Leu Glu Arg Lys Asp Gly Asp Glu Ile Asp
                85                  90                  95

Ala Met Leu Cys Gln Gln Ser Gly Phe Gly Pro Ser Ser Ile Lys Phe
            100                 105                 110

Arg Val

<210>  26
<211>  108
<212>  PRT
<213>  Arabidopsis thaliana

<400>  26

Met Val Ser Ser Thr Asp Thr Ile Ser Ala Ser Phe Val Ser Lys Lys
1               5                   10                  15

Ser Arg Ser Pro Glu Thr Ser Pro His Met Lys Val Thr Leu Lys Val
            20                  25                  30

Lys Asn Gln Gln Gly Ala Glu Asp Leu Tyr Lys Ile Gly Thr His Ala
        35                  40                  45

His Leu Lys Lys Leu Met Ser Ala Tyr Cys Thr Lys Arg Asn Leu Asp
    50                  55                  60

Tyr Ser Ser Val Arg Phe Val Tyr Asn Gly Arg Glu Ile Lys Ala Arg
65                  70                  75                  80

Gln Thr Pro Ala Gln Leu His Met Glu Glu Glu Asp Glu Ile Cys Met
                85                  90                  95

Val Met Glu Leu Gly Gly Gly Gly Pro Tyr Thr Pro
            100                 105